# EUROPEAN PATENT APPLICATION

(11) **EP 3 815 707 A1**
(43) Date of publication of application: **05.05.2021**
(21) Application number: 19826912.8
(22) Date of filing: 25.06.2019
(51) Int. Cl.: A61K 38/16, A61K 35/20, A61K 38/08, A61K 38/43, A61P 3/10, A61P 9/10, A61P 11/00, A61P 25/00, A61P 25/28, A61P 29/00, A61P 35/00, A61P 35/04

(54) **GLYCOSAMINOGLYCAN INHIBITOR AND PROMOTER**

(30) Priority: 26.06.2018 JP 2018121377
(71) Applicant: S&K Biopharma, Inc., Kawasaki-shi, Kanagawa 213-0012 (JP)
(72) Inventor: NAKAMURA Masao, Hachioji-shi, Tokyo 192-0982 (JP); SATO Atsushi, Fujisawa-shi, Kanagawa 251-0032 (JP); KAGAYA Shinji, Kawasaki-shi, Kanagawa 213-0012 (JP)
(74) Representative: HGF
(86) International application number: PCT/JP2019/025118
(87) International publication number: WO 2020/004378

(57) **Abstract**

The purpose of the present invention is to develop a medicine for diseases related to glycosaminoglycan functions with less side effects. Provided is a glycosaminoglycan inhibitor or promoter that comprises lactoferrin or a derivative thereof.

## Description

### TECHNICAL FIELD

The present invention relates to inhibitors and promoters of glycosaminoglycans. More specifically, the present invention relates to an inhibitor or promoter of glycosaminoglycan comprising lactoferrin or a derivative thereof as an active ingredient, and a medical use of lactoferrin in the treatment of glycosaminoglycan-related diseases based on the glycosaminoglycan inhibitory or promotive action of lactoferrin.

### BACKGROUND ART

Most glycosaminoglycans are in a form of proteoglycans, in which polysaccharides are added to a protein called a core protein, present in the extracellular matrix and cell surfaces of body tissues, and present as a major component of cartilage. Among others, chondroitin sulfate interacts with various cell growth factors and extracellular matrix components to regulate various cellular activities such as cell adhesion, migration, proliferation, differentiation, and morphogenesis. Moreover, medicines and food products made from sodium chondroitin sulfate are available on the market for the treatment of low back pain, joint pain, peri-articular inflammation of the shoulder, and so on. In the following, abbreviations are indicated as follows concerning sugars: chondroitin sulfate A (CS-A), chondroitin sulfate B (CS-B), chondroitin sulfate C (CS-C), chondroitin sulfate D (CS-D), chondroitin sulfate E (CS-E), hyaluronic acid (HA), heparin (HP), heparan sulfate (HS), and keratan sulfate (KS).

Excessive expression of chondroitin sulfate in inflammatory areas is associated with spinal cord injury, cancer metastasis, and Alzheimer's disease, leading to worsening of the disease. It is estimated that more than 100,000 people in Japan already suffer from spinal cord injuries in which chondroitin sulfate is involved in the improvement of the disease, with approximately 5,000 new cases each year, and approximately 200,000 new cases worldwide. The disease can be classified into an acute phase from the time of injury up to a few hours, a subacute phase up to a few weeks, and a chronic phase thereafter. While some degree of tissue repair and spontaneous recovery is achieved up to the subacute phase, after the chronic phase, the reconstruction of disrupted neural circuits is inhibited and recovery is difficult due to glial scars that have formed tightly at the site of injury (Non-Patent Document 1). Currently, recovery with high doses of steroids, hepatocyte growth factors, and regenerative medicine within 72 hours of injury has been tried, but no treatment has been established.

In spinal cord injury, reactive astrocytes, a major factor in glial scarring that inhibits the reconstruction of neural circuits, synthesize chondroitin sulfate proteoglycans. The receptors for glycosaminoglycans, PTPσ and LARs, are present on the surface of the growth cones of neurons at the site of injury, and it has been reported that chondroitin sulfate proteoglycans, especially CS-E glycans, are involved in the inhibition of axonal regeneration by binding to these receptors (Non-Patent Document 2). Therefore, in experiments using a spinal cord injury model, therapeutic effects have been reported with enhanced nerve regeneration by PTPσ of the CS-E receptor (Non-Patent Document 3) and knockout mice of the phosphatase LAR (Non-Patent Document 4), degradation of CS-E by the bacterial enzyme chondroitinase ABC, which degrades glycans that bind to the proteoglycan core protein (Non-Patent Documents 5, 6) and axonal regeneration by CS-E synthase inhibitors (Non-Patent Document 7).

In recent years, a new treatment method using chondroitinase ABC has been marketed for the treatment of lumbar disc herniation, with the promise of topical administration. However, because this product is a bacterial protein, side effects (including abnormalities in laboratory test results) were observed in 53.3% of cases in domestic clinical trials. The most common were Modic classification vertebral luminosity changes (23.6%), low back pain (22.3%), disc height reduction of more than 30% (14.4%), and lower extremity pain (4.8%), with the most serious ones being shock and anaphylaxis reported in the study. Thus, it is essential to develop drugs with fewer side effects.

In humans, there is an enzyme called hyaluronidase that degrades chondroitin sulfate. Hyaluronidase is not effective when administered to a dog model of spinal cord injury (Non-Patent Document 8), and when administered to a mouse model of spinal cord injury, no improvement in locomotion has been observed, but rather a tendency to worsen inflammation has been observed (Non-Patent Document 9). Treatment as regenerative medicine is also being considered, and in 2018, treatment with cultured autologous bone marrow mesenchymal stem cells has been approved. Attempts to regenerate damaged neurons from iPS cells have also been considered, but regenerative medicine has the disadvantage of being expensive to treat and taking a long time before the cells can be administered, making it impossible to respond to treatment at an early stage after an injury.

It has also been reported that chondroitin sulfate is highly expressed on the surface of cancer cells and has been implicated in cancer metastasis (Non-Patent Document 10). In particular, in lung cancer, cancer cells have been found to metastasize when RAGE proteins expressed in cells of normal lung tissue, especially in the vascular endothelium, bind to CS-E and other proteins (Non-Patent Documents 11 and 12). That metastasis is inhibited by chondroitinase ABC, anti-CS-E, and anti-RAGE antibodies. Glycosaminoglycans such as hyaluronic acid and CS-C have also been reported to bind to CD44, a surface marker of breast cancer stem cells involved in breast cancer metastasis (Non-Patent Document 10).

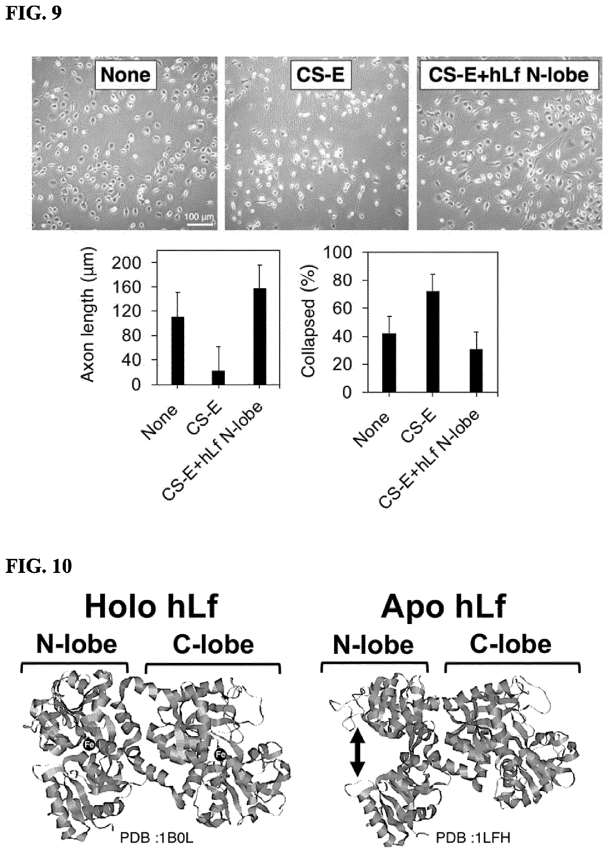

Lactoferrin (Lf), on the other hand, is an iron-binding glycoprotein with a molecular weight of about 80,000 that belongs to the transferrin family and is abundantly present in mammalian exocrine fluids such as colostrum and tears. Lactoferrin is composed of globular domains of N-lobe and C-lobe, and one trivalent iron is bound to each domain. The conformation is different between the iron-saturated (Holo) and iron-free (Apo) forms. The three-dimensional structure of human lactoferrin is shown in Figure 10.

Lactoferrin is a cell-secreted molecule that bridges innate and adaptive immune functions in mammals, and its protective effects range from anti-cancer, antiinflammatory, and immunomodulatory activities to antimicrobial activity against many microorganisms. This wide range of activities is reported to be made possible not only by the iron-binding capacity of Lf but also by a mechanism of action involving the interaction of Lf with molecular and cellular components of the host and pathogen (Non-Patent Document 13).

### BACKGROUD ART DOCUMENTS

### Non-Patent Documents

Non-Patent Document 1: J. Neurotrauma 2005; 22(5), 544-558
Non-Patent Document 2: Trends in Glycoscience and Glycotechnology 2011; 23(133) 201-211
Non-Patent Document 3: Science 2009 Oct 23; 326 (5952): 592-6
Non-Patent Document 4: Neurobiol Dis 2015; 73: 36-48
Non-Patent Document 5: FEBS Journal 280 (2013) 2462-2470
Non-Patent Document 6: Nature. 2002 Apr 11; 416 (6881): 636-40
Non-Patent Document 7: ACS Chem. Biol. 2017, 12, 3126-3133
Non-Patent Document 8: Surg. Neurol. 13, 157-159, 1980
Non-Patent Bibliography 9: Proceedings of the 37th Annual Meeting of the Japanese Society of Carbohydrate Science, Oda et al. August 30, 2018
Non-Patent Document 10: Biochemical Journal (2018) 475 587-620
Non-Patent Document 11: THE JOURNAL OF BIOLOGICAL CHEMISTRY 2008; 283(49): 34294-34304
Non-Patent Document 12: Biomed Res Int. 2013; 656319
Non-Patent Document 13: Biochimica et Biophysica Acta, Volume 1820, Issue 3, March 2012, Pages 226-236

### SUMMARY OF THE INVENTION

Therefore, there is a need for the development of medicines with fewer side effects for diseases related to the function of glycosaminoglycans.

As a means of overcoming the above shortcomings, the inventors propose a pharmaceutical composition with minimal side effects for diseases related to the function of glycosaminoglycans, typically using a safe human lactoferrin (hLF) and its functional moiety, derivatives, and the like, and a method of treating said diseases using the same.

In the search for biological substances that inhibit or enhance the function of glycosaminoglycans involved in the exacerbation of multiple diseases, the inventors have focused on lactoferrin and have examined whether lactoferrin inhibits or enhances the function of certain glycosaminoglycans. Using lactoferrin, its N-lobe, and the basic peptides contained in lactoferrin, the inventors have found that only certain glycosaminoglycans bind to lactoferrin by circular dichroism (CD) spectra, that pharmacological activity in ameliorating spinal cord injury is based on the axonal elongation activity of lactoferrin using a cellular model, and the cancer therapeutic effects include inhibition of cancer cell line growth, rate of cell death, and inhibition of cancer cell invasion and migration capacity by scratch assays. Based on these findings, they found that lactoferrin, and peptides, globular domains, mutants, and derivatives thereof having its activity could be effective therapeutic agents for diseases against certain glycosaminoglycans, and the present invention was completed.

The present disclosure includes the following [1] to [19].
[1] An inhibitor or promoter composition of glycosaminoglycans comprising lactoferrin or derivatives thereof.
[2] The inhibitor or promoter composition according to [1], wherein the lactoferrin is an iron-free (Apo) or iron saturated (Holo) lactoferrin.
[3] The inhibitor or promoter composition according to [1] or [2], wherein the lactoferrin is of human origin.
[4] The inhibitor or promoter composition according to [1], wherein the lactoferrin is a protein or peptide selected from the group consisting of (a) to (e) below.
   (a) a protein consisting of any one amino acid sequence of SEQ ID NOS: 1-6;
   (b) a protein comprising N-lobe of lactoferrin;
   (c) a protein consisting of the amino acid sequence in which 1 to 70 amino acids are deleted, substituted, inserted, and/or added in any one amino acid sequence of SEQ ID NOS: 1 to 6, and which has the activity to inhibit or promote the expression or function of chondroitin sulfate; and
   (d) a protein consisting of the amino acid sequence having 90% or more sequence identity to any one amino acid sequence of SEQ ID NOS: 1-6 and having activity that inhibits or promotes the expression or function of chondroitin sulfate; and
   (e) a peptide consisting of the amino acid sequence of GRRRRSVQWCA (SEQ ID NO: 7).
[5] The inhibitor or promoter composition according to any one of [1] to [4], the derivative of the lactoferrin is a hinge deletion human lactoferrin/human IgG Fc fusion protein (hLF-CH2-CH3) (SEQ ID NO: 9), human lactoferrin/human serum albumin fusion protein (hLF (-HSA) (SEQ ID NO: 10) or human lactoferrin-human granulocyte colony-stimulating factor (G-CSF) fusion protein (hLF-G-CSF) (SEQ ID NO: 11).
[6] The inhibitor or promoter composition according to any one of [1] to [5], wherein the glycosaminoglycan is any one selected from the group consisting of chondroitin sulfate-A (CS-A), chondroitin sulfate-B (CS-B), chondroitin sulfate-C (CS-C), chondroitin sulfate-D (CS-D), chondroitin sulfate-E (CS-E), heparin (HP) and heparan sulfate (HS), and keratan sulfate (KS).
[7] The inhibitor or promoter composition according to [6], wherein the glycosaminoglycan is chondroitin sulfate-C (CS-C), chondroitin sulfate-D (CS-D), or chondroitin sulfate-E (CS-E).
[8] The inhibitor or promoter composition according to [6], wherein the glycosaminoglycan is CS-E.
[9] A pharmaceutical composition for treating a glycosaminoglycan-related disease or condition, comprising lactoferrin or a derivative thereof.
[10] The pharmaceutical composition according to [9], wherein the lactoferrin is iron-free (Apo) or iron saturated (Holo) lactoferrin, prepared in an amount from 0.001 to 10 g/kg/day.
[11] The pharmaceutical composition according to [9] or [10], wherein the lactoferrin is of human origin.
[12] The pharmaceutical composition according to [9], wherein the lactoferrin is a protein or peptide selected from the group consisting of (a) to (e) below:
   (a) a protein consisting of any one amino acid sequence of SEQ ID NOS: 1-6;
   (b) a protein comprising N-lobe of lactoferrin;
   (c) a protein consisting of the amino acid sequence in which 1 to 70 amino acids are deleted, substituted, inserted, and/or added in any one amino acid sequence of SEQ ID NOS: 1 to 6, and which has the activity to inhibit or promote the expression or function of glycosaminoglycans; and
   (d) a protein consisting of the amino acid sequence having 90% or more sequence identity to any one amino acid sequence of SEQ ID NOS: 1-6 and having activity that inhibits or promotes the expression or function of chondroitin sulfate; and
   (e) a peptide consisting of the amino acid sequence of GRRRRSVQWCA (SEQ ID NO: 7).
[13] The pharmaceutical composition according to any one of [9] to [12], wherein the derivative of the lactoferrin is a hinge deletion human lactoferrin/human IgG Fc fusion protein (hLF-CH2-CH3) (SEQ ID NO: 9), human lactoferrin/human serum albumin fusion protein (hLF-HSA) (SEQ ID NO: 10) or human lactoferrin-human granulocyte colony-stimulating factor (G-CSF) fusion protein (hLF-G-CSF) (SEQ ID NO: 11).
[14] The pharmaceutical composition according to any one of [9] to [13], wherein the glycosaminoglycan is chondroitin sulfate-C (CS-C), chondroitin sulfate-D (CS-D), or chondroitin sulfate-E (CS-E).
[15] The pharmaceutical composition according to any one of [9] to [14], for use in combination with chondroitin sulfate-degrading enzymes.
[16] The pharmaceutical composition according to any one of [9] to [15], wherein the disease is any one selected from the group consisting of spinal cord injury, cancer metastasis, cancer, diabetes, inflammation, atherosclerosis, fibrosis, acute respiratory disease, and Alzheimer's disease.
[17] The pharmaceutical composition according to any one of [9] to [16], which is in the form of a food product.
[18] The pharmaceutical composition according to any one of the [9] to [16], which is in the form of an injection.
[19] The pharmaceutical composition according to any one of [9] to [16], wherein the pharmaceutical composition is for an oral administration.

The inventors have found that hLF, hLF N-lobe, and their derivatives, hLF-CH2-CH3, hLF-HSA, hLF-G-CSF bind strongly to CS-E, a glycosaminoglycan. In experiments with chick embryo spinal dorsal root ganglion neurons, CS-E inhibition of nerve axon outgrowth and disruption of growth cones were observed, while it was observed that the addition of hLF, hLF N-lobe, hLF-CH2-CH3, hLF-HSA, and hLF-G-CSF resulted in inhibition of axonal elongation and protection against disruption of growth cones. This indicated their potential for therapeutic applications, especially in the acute phase.

A glycosaminoglycan inhibitor/promoter composition containing lactoferrin or a derivative thereof is useful as a medicine for the treatment of diseases or conditions related to the function of glycosaminoglycans.

### BRIEF DESCRIPTION OF THE DRAWINGS

Figure 1 shows the results of secondary structure analysis of (Apo and Holo) full-length human lactoferrin (hLf) with different degrees of iron-binding in the presence of various glycosaminoglycans by CD spectroscopy. (A) Results of secondary structure analysis of iron-saturated hLf (Holo hLf); (B) Results of secondary structure analysis of iron-free hLf (Apo hLf).
Figure 2 shows the results of secondary structure analysis of hLf N-lobe with different degrees of iron-binding in the presence of various glycosaminoglycans by CD spectroscopy. (A) Secondary structure analysis of the iron-saturated hLf N-lobe (Holo hLf N-lobe); (B) Secondary structure analysis of the iron-free hLf N-lobe (Apo hLfN-lobe).

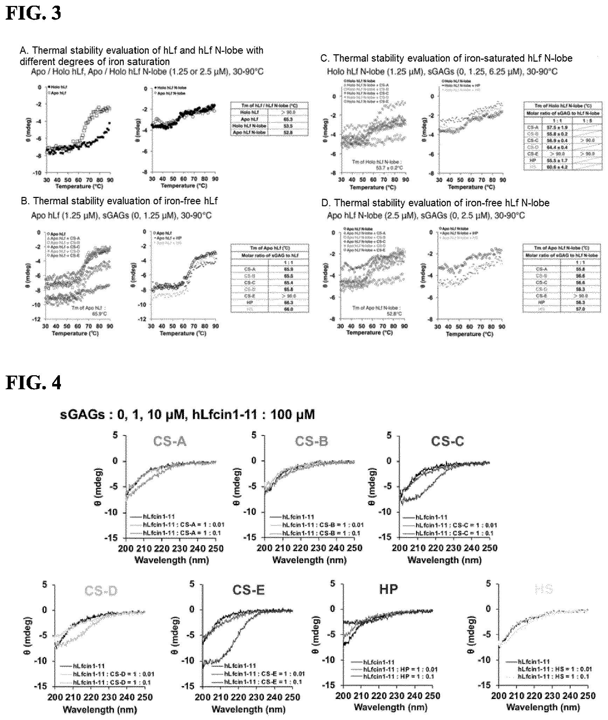

Figure 3 shows the results of thermal denaturation studies of full-length human lactoferrin (hLf) and hLf N-lobe with different degrees of iron-binding in the presence of various glycosaminoglycans, respectively, by CD spectroscopy. (A) Thermal stability of Apo/Holo hLf (left) and Apo/Holo hLf N-lobe (right) in the absence of glycosaminoglycans is shown. (B) Thermal stability of Apo hLf in the presence of various glycosaminoglycans (CS-A to E (left), HP, and HS (right)). (C) Thermal stability of Holo hLf N-lobe in the presence of various glycosaminoglycans (CS-A to E (left), HP, and HS (right)). (D) Thermal stability of Apo hLf N-lobe in the presence of various glycosaminoglycans (CS-A to E (left), HP, and HS (right)).
Figure 4 shows the results of secondary structure analysis of hLfcin1-11 (GRRRRSVQWCA (SEQ ID NO: 7)) in the presence of various glycosaminoglycans by CD spectroscopy.
Figure 5 is a graph showing the effect of hLF N-lobe on cell proliferation of chlorate-treated (NaClO₃)-treated non-small cell lung cancer cell line PC-3 in the presence of various glycosaminoglycans (CS-A to E).
Figure 6 is a diagram showing the effect of iron-saturated (Holo) hLF N-lobe on cell proliferation of (A) chlorate (NaClO₃) or (B) chondroitin sulfate-degrading enzyme (ChABC)-treated non-small cell lung cancer cell line PC-3.
Figure 7 is a diagram showing the effect of hLF N-lobe on CS-E-dependent metastasis of human lung cancer PC-14 cells.
Figure 8 is a diagram showing the effect of various glycosaminoglycans (CS-A to E) on protrusion stretching and growth cones of PC-12 cells.
Figure 9 is a diagram showing the effects of hLf N-lobe on the protrusion elongation and growth cones of PC-12 cells in the presence of various glycosaminoglycans (CS-A to E).
Figure 10 is a diagram showing the three-dimensional structure of human lactoferrin (background art).
Figure 11 is a diagram showing the results of binding analysis of human lactoferrin (hLF), hLF N-lobe, and hLF derivatives (hLF-CH2-CH3, hLF-HSA, hLF-G-CSF) with various sulfated glycosaminoglycans (sGAGs) by solid-phase binding assay.
Figure 12 is a diagram showing the inhibitory effects of hLF, hLF N-lobe, and hLF derivatives (hLF-CH2-CH3, hLF-HSA, and hLF-G-CSF) on the axonal outgrowth inhibition activity of CS-E in chick embryo spinal cord dorsal root ganglion (DRG) cells.
Figure 13 is a diagram showing the results of the inhibitory effects of hLF, hLF N-lobe, and hLF derivatives (hLF-CH2-CH3, hLF-HSA, hLF-G-CSF) on the growth cone collapse activity of CS-E chick embryo spinal cord DRG cells.

### EMBODIMENTS FOR IMPLEMENTING THE INVENTION

### 1. Definition of terms

The term "lactoferrin" as used herein means full-length lactoferrin, a protein consisting of a functional moiety of lactoferrin, or a protein containing such a functional moiety, unless otherwise stated. As used herein, "functional moiety" of lactoferrin means a portion of lactoferrin or a variant(s) of lactoferrin that by itself has a function equivalent to or at least partly equivalent to that of full-length lactoferrin. Full-length lactoferrin includes but are not limited to human lactoferrin (SEQ ID NO: 1), bovine lactoferrin (SEQ ID NO: 2), sheep lactoferrin (SEQ ID NO: 3), goat lactoferrin (SEQ ID NO: 4), horse lactoferrin (SEQ ID NO: 5), and camel lactoferrin (SEQ ID NO: 6) and others.

Information on the amino acid sequences of these lactoferrins and the nucleotide sequences encoding them is available by accessing sequence databases (e.g., the NCBI database) that are readily accessible on the Web. For convenience, Table 1 below shows these human and some mammalian sequence information.

**[Table 1]**

| Origin of lactoferrin | Genbank Accession No. | SEQ ID NO: |
|---|---|---|
| Human | ANC33015.1 | 1 |
| Bovine | NP_851341.1 | 2 |
| Sheep | ACT76166.1 | 3 |
| Goat | AAA97958.1 | 4 |
| Horse | NP_001157446.1 | 5 |
| Camel | AAF82241.1 | 6 |

The term "function of lactoferrin" herein means the ability to inhibit or promote the expression or function of a glycosaminoglycan, at least *in vitro* and preferably *in vivo.* The "function of the glycosaminoglycan" includes any function of the glycosaminoglycan that is recognized by those skilled in the art as a function of the glycosaminoglycan, including, but not limited to, the regulation of various cellular activities such as cell adhesion, migration, proliferation, differentiation, and morphogenesis.

The "functional part" of lactoferrin typically includes
(1) the globular domain of lactoferrin (N-lobe or C-lobe);
(2) lactoferrin variants (e.g., a protein consisting of an amino acid sequence with 90% or more identity to any of the amino acid sequences of SEQ ID NOS: 1-6, or an amino acid sequence having substitutions, deletions, insertions, and/or additions of one to several amino acid residues (e.g., from two to about 70) in any of the amino acid sequences of SEQ ID NOS: 1-6);
(3) a partial peptide of lactoferrin (e.g., a peptide consisting of amino acid residues 20-30 of human lactoferrin ("GRRRRSVQWCA" (SEQ ID NO: 7)); and the like.

The variants mentioned in (2) above are typically, but not limited to, variants of lactoferrin having any one amino acid sequence of naturally occurring SEQ ID NOS: 1-6, and include those that can be acquired artificially using site-specific mutagenesis methods described in, for example, Manual Vol. 3, Cold Spring Harbor Laboratory Press 2001", "Ausubel, Current Protocols in Molecular Biology, John Wiley & Sons 1987-1997", "Nuc. Acids. Res., 10, 6487 (1982)", "Proc. Natl. Acad. Sci. USA, 79, 6409 (1982)", "Gene, 34, 315 (1985)", "Nuc. Acids. Res., 13, 4431 (1985)", "Proc. Natl. Acad. USA, 82, 488 (1985)" and others.

In the example of the variants of (2) above, the degree of mutation by substitutions, deletions, insertions, and/or additions of amino acid residues in the amino acid sequence is permissible as long as the variant protein has the same or at least some of the functions of the original protein, and the degree can be typically up to about 10% of the original amino acid sequence, i.e., from one to several (e.g., from two to about 70 sequentially) in the number of amino acid residues.

In the example of the variants in (2) above, "90% or more identity" may include 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, and 99.9% or more sequence identity to the original amino acid sequence.

As used herein, "derivative(s) of lactoferrin" means a lactoferrin molecule with a desired modification to lactoferrin by genetic engineering techniques. Examples of such a derivative(s) of lactoferrin include, for example, a fusion protein of lactoferrin with another protein. Examples of such fusion proteins include a hinge deletion human lactoferrin/human IgG Fc fusion protein (hLF-CH2-CH3) (SEQ ID NO: 9), human lactoferrin/Human serum albumin fusion protein (hLF-HSA) (SEQ ID NO: 10), human lactoferrin-human granulocyte colony-stimulating factor (G-CSF) fusion protein (hLF-G-CSF) (SEQ ID NO: 11), etc.

For lactoferrin, "ability to inhibit or promote the expression or function of glycosaminoglycans" can be quantitatively measured by comparing measures of glycosaminoglycan expression or function in the absence and presence of lactoferrin, and verifying that the above measures are reduced or increased in the presence of lactoferrin. Non-limiting examples of such quantitative measurement methods are shown in the examples described later in this specification.

As used herein, "glycosaminoglycan" is used in the sense generally recognized by those skilled in the art and refers to a long non-branched polysaccharide comprising a repeating disaccharide unit. Examples of glycosaminoglycans concerning the present invention include but are not limited to chondroitin sulfate A (CS-A), chondroitin sulfate B (CS-B), chondroitin sulfate C (CS-C), chondroitin sulfate D (CS-D), chondroitin sulfate E (CS-E), hyaluronic acid (HA), heparin (HP), heparan sulfate (HS), keratan sulfate (KS), etc.

### 2. Glycosaminoglycan inhibitors or accelerators containing lactoferrin

According to one embodiment of the invention, there is provided a glycosaminoglycan inhibitor- or promoter-composition (or a composition for inhibition or promotion of glycosaminoglycans) containing lactoferrin. In the above inhibitor or promoter composition, the lactoferrin can be iron-free (Apo) or iron saturated (Holo) lactoferrin. The lactoferrin-containing glycosaminoglycan inhibitor or promoter composition of the present invention exploits the ability of lactoferrin to inhibit or promote the expression or function of glycosaminoglycans, and the glycosaminoglycan inhibitor or promoter composition of the present invention enables the expression or function of glycosaminoglycans is inhibited or facilitated.

The glycosaminoglycan inhibitor or promoter composition of the present invention may include, in addition to lactoferrin, any solvent, dispersant, binder, additive, or other excipient known in the art.

Glycosaminoglycan Inhibitor or promoter composition containing lactoferrin of the present invention can be used *in vitro* or *in vivo.*

### 3. Application of glycosaminoglycan inhibitor or promoter composition containing lactoferrin in pharmaceuticals

According to a further embodiment of the present invention, a pharmaceutical composition for treating a disease or condition related to glycosaminoglycans (hereinafter simply referred to as "the pharmaceutical composition of the present invention") containing lactoferrin is provided. In the pharmaceutical compositions of the present invention, the lactoferrin is iron-free (Apo) or iron saturated (Holo) and is typically prepared to have an amount of lactoferrin between 0.001 and 10 g/kg/day. The subject may be a human or an animal (e.g., a mammal other than a human). The pharmaceutical compositions of the present invention can also be used in combination (concurrently or continuously) with other agents (e.g., chondroitin sulfate-degrading enzymes).

Since lactoferrin is a protein originally present in living organisms and also in foods, it has the advantage that when used as a medicine according to the present invention, it has fewer side effects, does not cause pain to the patient or the subject, and has less risk of inducing other diseases.

Examples of "diseases or conditions associated with glycosaminoglycan function" typically include, but are not limited to, cancer (e.g., lung, breast, testicular, liver, etc.), cancer metastases, diseases associated with axonal regeneration (e.g., spinal cord injury, Alzheimer's disease, etc.), diabetes, inflammation, atherosclerosis, fibrosis, and acute respiratory disease.

In the pharmaceutical compositions of the present invention, the lactoferrin can be formulated as it is, or formulated together with a pharmaceutically acceptable carrier or the like as a medicine and administered to humans or animals for the treatment of various diseases. Accordingly, the present invention also provides a method of treating a disease or condition associated with (a function of) glycosaminoglycans, including the step of administering the lactoferrin to a patient or subject in need of treatment.

According to a further embodiment of the invention, lactoferrin can be used for the manufacture of pharmaceuticals for the treatment of diseases or conditions associated with glycosaminoglycans.

In the pharmaceutical compositions of the present invention, the lactoferrin can be provided in a kit included in said kit as one of the components of a medical (e.g., for treatment or prevention) kit. The kit may include said pharmaceutical composition as well as instructions for use describing the method of application, the amount to be applied, etc.

The term "pharmaceutically acceptable" or "pharmacologically acceptable" is used herein to describe such substances, materials, compositions, and/or dosage forms that are free from excessive toxicity, irritation, allergic reactions, or other problems or complications, are commensurate with a reasonable benefit/hazard ratio, and are within the correct medical judgment of being appropriate for use in contact with human and animal tissue.

As used herein, "pharmaceutically (or pharmacologically) acceptable carriers" include any solvent, dispersant, coating, surfactant, antioxidant, preservative (e.g., antimicrobial, antifungal), isotonic agent, absorption retarder, salt, preservative, drug, drug, drug stabilizer, gel, binder, additive, disintegrant, lubricant, sweetener, flavoring agent, dye, and/or materials and combinations thereof known in the art.

As used herein, "pharmaceutically (or pharmacologically) acceptable carriers" include any solvent, dispersant, coating, surfactant, surfactant, antioxidant, preservative (e.g., antimicrobial, antifungal), isotonic agent, absorption retarder, salt, preservative, drug, drug, drug stabilizer, gel, binder, additive, disintegrant, lubricant, sweetener, flavoring agent, dye, and/or materials and combinations thereof known in the art.

As used herein, "treatment" or "treating" includes (i) preventing a pathological condition from occurring (e.g., prevention), (ii) blocking the pathological condition or its development, (iii) reducing, remitting, or curing the pathological condition and/or reducing, remitting, or curing the pathological condition and associated symptoms.

When lactoferrin is administered to a patient or subject as a medicine according to the present invention, it can be formulated, e.g., lactoferrin alone or with customary excipients, and used for oral or parenteral administration as capsules, tablets, injections, or other appropriate dosage forms. For example, the capsules can be prepared by mixing the inhibitor or promoter composition or lactoferrin of the present invention with excipients such as lactose, starch or derivatives thereof, cellulose derivatives, etc., and filling the gelatin capsules.

Besides the above excipients, the tablets can be prepared into tablets using an ordinary compression tableting machine by adding binders such as sodium carboxymethylcellulose, alginate, gum arabic, and the like, and water to knead the tablets into granules, if necessary, and then adding a lubricant such as talc or stearic acid.

Moreover, for parenteral administration by injection, lactoferrin is dissolved in sterile distilled water or sterile saline, together with a dissolution aid, and sealed in an ampoule to make a formulation for injection. If necessary, stabilizers, buffering substances, and the like may be added. These parenteral preparations may be administered intravenously or by intravenous infusion.

Furthermore, lactoferrin may be added to nutritional supplements, food, and beverages, etc., either as is or after being formulated.

According to the present invention, the dose of lactoferrin varies according to various factors, such as symptoms, severity, age, and presence of complications of the patient or subject to be treated. It also varies depending on the route of administration, dosage form, number of doses, etc., but in general, when administered orally, the dosage of lactoferrin can usually be 0.001 to 10 g/kg/day, 0.005 to 10 g/kg/day, 0.01 to 10 g/kg/day, and 0.01 to 5 g/kg/day, as an active ingredient. When administered to humans, typically, the therapeutically effective doses are 10 mg to 15,000 mg, 10 mg to 12,000 mg, 10 mg to 10,000 mg, 20 mg to 10,000 mg, 20 mg to 8,000 mg, 30 mg to 8,000 mg, and 30 mg to 6,000 mg per day. Such a daily dose can be administered in a single or divided dose per day to patients in need of treatment of a disease or condition related to glycosaminoglycans. In the case of parenteral administration, the dose can be selected and administered in the range of about 1/100 to 1/2 of the dose in the case of oral administration, as appropriate.

The present invention is described in more detail by the following examples. These examples are illustrative only and do not limit the scope of the invention in any manner.

### EXAMPLES

Examples are given below. Abbreviations in the text and the figures are human lactoferrin (hLF), human lactoferrin N-lobe (hLF N-lobe), hinge deletion human lactoferrin/human IgG Fc fusion protein (hLF-CH2-CH3), human lactoferrin/human serum albumin fusion protein (hLF-HSA), human lactoferrin-human granulocyte colony-stimulating factor (G-CSF) fusion protein (hLF-G-CSF), human IgG (hIgG), human serum albumin (HSA), chondroitin sulfate A, B, C, D, E (CS-A, B, C, D, E), heparin (HP), sulfated glycosaminoglycan (sGAG) and the chick embryo spinal cord dorsal root ganglion (DRG).

### Example 1: Secondary Structure and Evaluation of Thermal Stability of Full-length Human Lactoferrin (hLf) and hLf Spherical Domains in the Presence of Various Glycosaminoglycans

The molar ratios of hLf, hLf N-lobe, and sGAG glycans were set to 1:0, 1:1, and 1:5, and the total liquid volume was prepared to 250 µl in PBS(-). The prepared samples were incubated in an aluminum block at 37°C for 1 hour. A circular dichroism dispersion meter J-1500 (JASCO) is shown in Tables 2-1 and 2-2.

### [Table 2]

**Table 2-1. Measurement Conditions for Secondary Structure Evaluation**

| | |
|---|---|
| CD Scale | 200 mdeg/1.0 dOD |
| FL Scale | 200 mdeg/1.0 dOD |
| Response | 1 sec |
| Band Width | 1.00 nm |
| Starting Wavelength | 250 nm |
| End Wavelength | 200 nm |
| Data Capture Interval | 0.1 nm |
| Scanning Mode | continuous |
| Scanning Speed | 20 nm/min |
| Stacking Times | 3 |
| Temperature in the holder | 37°C |

**Table 2-2. Measurement Conditions for Circular Dichroism Dispersion Meter**

| | |
|---|---|
| CD scale | 200 mdeg/1.0 dOD |
| FL Scale | 200 mdeg/1.0 dOD |
| Response | 1 sec |
| Bandwidth | 1.00 nm |
| Measurement Wavelength | 225 nm |
| Starting Temperature | 30°C |
| End Temperature | 90°C |
| Data Capture Interval | 1°C |

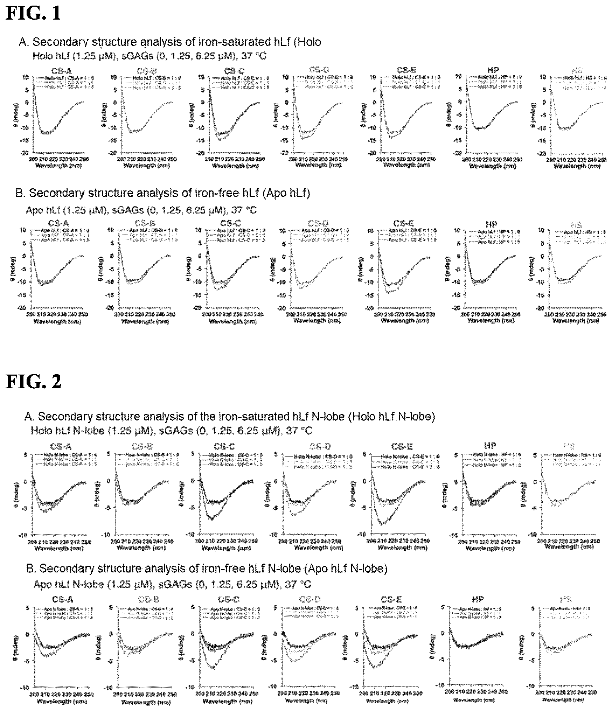

Figure 1 shows the results of secondary structure analysis of (Apo and Holo) full-length human lactoferrin (hLf) with different degrees of iron-binding in the presence of various glycosaminoglycans by CD spectroscopy. Figure 1A shows the results of secondary structure analysis of iron-saturated hLf (Holo hLf) and Figure 1(B) shows the results of secondary structure analysis of iron-free hLf (Apo hLf). In the presence of CS-C, CS-D, and CS-E, a remarkable change in the secondary structure of full-length hLf was observed. This effect was not affected by the presence or absence of iron in hLf.

Figure 2 shows the results of secondary structure analysis of hLf N-lobe with different degrees of iron-binding in the presence of various glycosaminoglycans by CD spectroscopy. Figure 2A shows the results of secondary structure analysis of the iron-saturated hLf N-lobe (Holo hLf N-lobe), and Figure 2B shows the results of secondary structure analysis of the iron-free hLf N-lobe (Apo hLf N-lobe). The results of the structural analysis show that the secondary structure of hLf N-lobe was significantly altered in the presence of CS-C, CS-D, and CS-E. This effect was not affected by the presence or absence of iron in the hLf N-lobe.

Figure 3 shows the results of thermal denaturation studies of full-length human lactoferrin (hLf) and hLf N-lobe, respectively, with different degrees of iron-binding in the presence of various glycosaminoglycans by CD spectroscopy. Figure 3A shows the thermal stability of Apo/Holo hLf (left) and Apo/Holo hLf N-lobe (right) in the absence of glycosaminoglycans. Figure 3B shows the thermal stability of Apo hLf in the presence of various glycosaminoglycans (CS-A to E (left), HP, and HS (right)). Figure 3C shows the thermal stability of Holo hLf N-lobe in the presence of various glycosaminoglycans (CS-A to E (left), HP, and HS (right)). Figure 3D shows the thermal stability of Apo hLf N-lobe in the presence of various glycosaminoglycans (CS-A to E (left), HP, and HS (right)). The thermal stability of full-length hLf and hLf N-lobe, in the presence of CS-E, was significantly improved. This effect was not affected by the presence or absence of iron in hLf and hLf N-lobe.

Figure 4 shows the results of secondary structure analysis of hLfcin1-11 (GRRRRSVQWCA) in the presence of various glycosaminoglycans by CD spectroscopy. The secondary structure of hLfcin1-11 was altered in the presence of CS-C, CS-D, and CS-E.

### Example 2: Activity of Iron-Saturated Human Lactoferrin N-lobe against PC-3 Treated with Sodium Chlorate (NaClO₃)

### 2-1. Cell proliferation test

Sodium chlorate (NaClO₃) (Wako) was dissolved in ultrapure water to a final concentration of 1 M. The prepared solution was filter sterilized using a 0.22 µm syringe filter. Sodium chlorate treatment has been reported to inhibit the sulfation of their glycans to cells (Hoogewerf et al., The Journal of Biological Chemistry, 266, 16564-16571 (1991)). The present evaluation was used to assess whether cell surface glycans are involved in the activity of the N-lobe.

96-well plates were coated with gelatin. The gelatin solution was discarded and cells were seeded at 5 × 10³ cells/well in the presence of 1 mM sodium chlorate and incubated in a 37°C 5% CO₂ incubator for 24 hours. As a control, cells seeded in the absence of sodium chlorate were prepared and cultured in the same manner. The sodium chlorate solution was adjusted using RPMI-1640 containing 5% FBS to a final concentration of 1 mM. The supernatant was discarded and replaced with a test medium; the cells were incubated in a 37°C 5% CO₂ incubator for 3 days. 10 µl/well of Cell Counting kit-8 was added and allowed to develop color for 2 hours in a 37°C 5% CO₂ incubator. The absorbance was measured at 450 nm using a microplate reader.

96-well plates were coated with gelatin. The gelatin solution was discarded and cells were seeded at 5 × 10³ cells/well in the presence of 1 mM sodium chlorate and cultured in a 37°C 5% CO₂ incubator for 24 hours. As a control, cells seeded in the absence of sodium chlorate were prepared and cultured in the same manner. Sodium chlorate and N-lobe were adjusted with RPMI-1640 containing 5% FBS to a final concentration of 1 mM and 5 µM, respectively. Sodium chlorate was adjusted to a final concentration of 1 mM using RPMI-1640 with 5% FBS. N-lobe was adjusted using RPMI-1640 with 5% FBS to a final concentration of 5 µM. RPMI-1640 containing 5% FBS was prepared as a control. The culture supernatant was discarded and 100 µl/well of test medium was added to the culture supernatant; it was incubated in a 37°C 5% CO₂ incubator for 3 days. 10 µl/well of Cell Counting kit-8 was added and allowed to develop color for 2 hours in a 37°C 5% CO₂ incubator. The absorbance was measured at 450 nm using a microplate reader. If the absorbance was greater than 1, dilutions were made and measurements were performed.

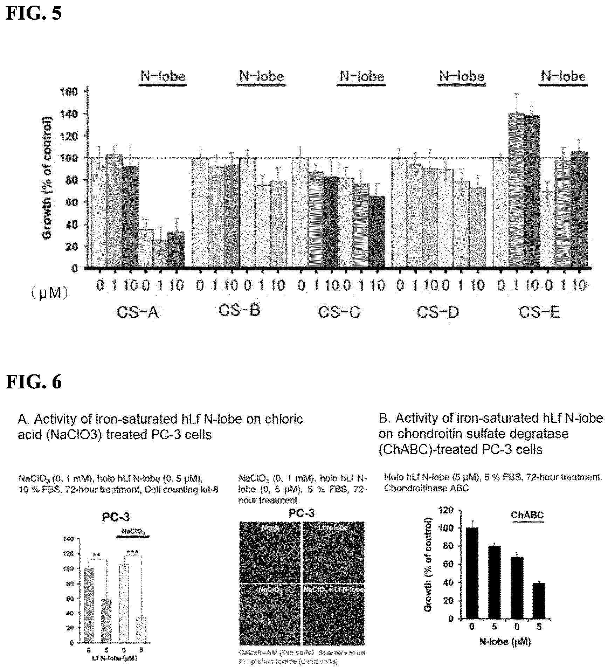

Figure 5 shows the effect of hLf N-lobe on cell proliferation of chlorate (NaClO₃)-treated non-small cell lung cancer cell line PC-3 in the presence of various glycosaminoglycans (CS-A to E). The presence of CS-C and CS-D enhanced the inhibition of cell proliferation of hLf N-lobe on PC-3 cells, and CS-E-induced cell proliferation was inhibited by the presence of N-lobe.

### 2-2. Cell survival test

Glass bottom dishes (IWAKI) were coated with 0.1% gelatin. The gelatin solution was discarded and PC-3 cells were seeded at 5 × 10⁴ cells/dish and incubated in a 37°C 5% CO₂ incubator for 24 h. 10 µM N-lobe solution was adjusted to 10 µM with RPMI-1640 containing 10% FBS. As a control' 0 µM, the same amount of PBS(-) was added as when 10 µM of N-lobe solution was added to the serum-containing medium. The cells were incubated in a 37°C 5% CO₂ incubator for 3 days. 1 mg/ml PI Solution (Dojin Chemical) was added at a final concentration of 1 µg/ml, 4 mM Calcein-AM (Thermo scientific) was dissolved in RPMI-1640 at a final concentration of 400 µM to make the staining solution.

For PC-3 cells, the following procedure was followed. The supernatant was discarded and 300 µl/dish of serum-free RPMI-1640 was added and the cells were washed. The washing solution was discarded and 200 µl/dish of staining solution was added at 200 µl/dish and the cells were incubated in a 37°C 5% CO₂ incubator for 15 minutes, while shielded from light. The supernatant was discarded and serum-free RPMI-1640 was added at 300 µl/dish and washed. For PC-3 cells, the following was performed. The supernatant was collected in 1.5 ml tubes and the cells remaining in the dish were added with 300 µl/dish of serum-free RPMI-1640. The collected supernatant was centrifuged at 200 x g, 4°C for 2 min. The supernatant was discarded and tapped with 500 µl/tube of serum-free RPMI-1640 and centrifuged at 200 x g for 2 minutes at 4°C. The supernatant was discarded and 100 µl/tube of staining solution was added and tapped. The supernatant of the dish was discarded and 200 µl/dish of staining solution was added at 200 µl/dish. All cells collected in the tubes were also returned to the dish with the stain solution, and the cells were incubated in a 37°C 5% CO₂ incubator for 15 min, while shielded from light. The supernatant was collected in 1.5 ml tubes, and serum-free RPMI-1640 was added to the remaining cells in the dish at 300 µl/dish. The collected supernatant was centrifuged at 200 x g, 4°C for 2 min. The supernatant was discarded and 1 ml/tube of serum-free RPMI-1640 was added to the supernatant and tapped. This operation was performed three times as a washing operation. The washing solution was discarded and RPMI-1640 without serum was added at 200 µl/dish. The supernatant of the dish was discarded and 200 µl/dish of serum-free RPMI-1640 was added at 200 µl/dish. The entire amount of cells collected in the tubes was also returned to the dish. The cells were observed using a scanning confocal laser microscope FV3000RS.

Figure 6(A) shows the effect of iron-saturated (Holo)hLF N-lobe on cell proliferation of chlorate (NaClO₃)-treated non-small cell lung cancer cell line PC-3. A decrease in the number of viable cells and an increase in dead cells were observed with N-lobe treatment in chlorate-treated PC-3 cells, suggesting that sugars present on the cell surface attenuate the activity of the N-lobe.

### 2-3. Activity of iron-saturated human lactoferrin N-lobe on chondroitin sulfate degrading enzyme (ChABC)-treated PC-3 cells

PC-3 cells were seeded at 5 × 10³ cells/well and cultured in the presence of 1 UN/ml chondroitin sulfate ChABC (SIGMA) for 24 hours in a 5% CO₂ incubator at 37°C. As a control, cells seeded in the absence of 1 UN/ml ChABC were prepared and cultured in the same manner. When the cells were seeded, RPMI-1640 containing 5% FBS was used; RPMI-1640 containing 5% FBS, 1 UN/ml ChABC, and 5 µM N-lobe and RPMI-1640 containing 5% FBS, 1 UN/ml ChABC, was prepared. RPMI-1640 containing 5% FBS, 5 µM N-lobe, and RPMI-1640 containing 5% FBS, were also prepared. The culture supernatant was discarded and 100 µl/well of test medium was added to the culture supernatant; it was incubated in a 37°C 5% CO₂ incubator for 3 days. 10 µl/well of Cell Counting kit-8 was added and allowed to develop color for 2 hours in a 37°C 5% CO₂ incubator. The absorbance was measured at 450 nm using a microplate reader.

Figure 6(B) shows the effect of iron-saturated (Holo) hLF N-lobe on the cell proliferation of chondroitin sulfate-degrading enzyme (ChABC)-treated non-small cell lung cancer cell line PC-3. In ChABC-treated PC-3 cells, the inhibition of growth by N-lobe was more enhanced, confirming that CS on the cell surface attenuates the activity of N-lobe.

Chlorate (NaClO₃) treatment alone had no particular effect on cell proliferation (Figure 6(A)). In contrast, treatment with chondroitin sulfuric acid degrading enzyme (ChABC) alone inhibited the cell proliferation of PC-3 cells (Figure 6(B)). This indicates that the inhibition of cell proliferation of PC-3 cells by ChABC treatment plus N-lobe was enhanced, i.e., at least an additive effect of the combination of ChABC treatment and N-lobe was confirmed.

### Example 4: Effect of the Human Lactoferrin N-Lobe on the CS-E-Dependent Metastasis of Human Lung Cancer Cells, PC-14 Cells

PC-14 cells were seeded in 12-well plates at 4 x 10⁵ cells/well and cultured overnight to form a cell monolayer. Uniform wounds were formed on the cell monolayer with a scratcher while applying a scratch guide. Serum-free RPMI-1640 was added at 1 ml/well and washed. The washing operation was performed three times. rhLF solution or N-lobe solution was adjusted to 5 µM with RPMI-1640 containing 10% FBS and incubated at 37°C. The culture supernatant was discarded and 400 µl/well of rhLF solution or N-lobe solution-containing medium was added. As a control, the same amount of PBS(-) medium was added at 400 µl/well as the amount of rhLF and N-lobe solutions added.The cultures were incubated overnight in a 5% CO₂ incubator at 37°C. The cells' supernatant after incubation was discarded and a 4% formaldehyde solution warmed to 37°C was added at 350 µl/well. The cells were kept at room temperature for 20 minutes to immobilize the cells. The supernatant was discarded and 1 ml/well of PBS(-) warmed to 37°C was added at 1 ml/well and left to stand at room temperature for 10 minutes. This operation was performed three times as a washing operation. The washing solution was discarded, and 350 µl/well of staining solution (0.2% crystal violet with 2% ethanol) was added at 350 µl/well and left to stand for 15 minutes at room temperature. The staining solution was discarded and PBS(-) was added at 1 ml/well and allowed to stand for 10 min at room temperature. This operation was performed four times as a washing operation. The stained cells were observed under a microscope. The distances of the wounds were analyzed using Image J.

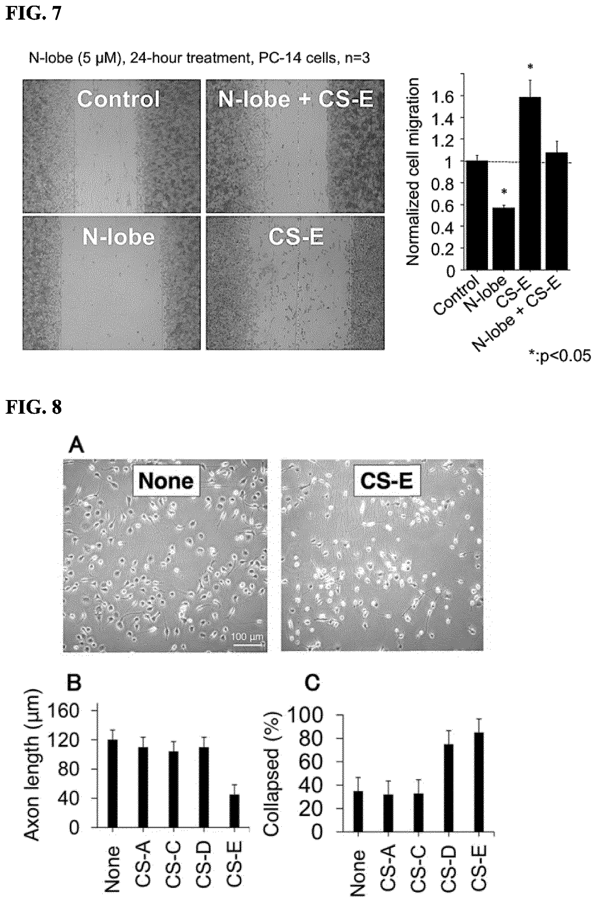

Figure 7 shows the effect of hLF N-lobe on the CS-E-dependent metastasis of human lung cancer PC-14 cells. Evaluated using scratch assay: PC-14 cells metastasized more in the presence of CS-E (assessed by the number of cells present inside the scratch). The addition of N-lobe reduced the number of cells that metastasized in the presence of CS-E. This indicates that hLf N-lobe inhibited the CS-E-dependent metastasis of PC-14 cells.

### Example 5: Effect on Spinal Cord Injury Models Using PC-12 Cells 5-1. Spinal cord injury model using CS-E in PC-12 cells

To measure the neurite length of the rat adrenal pheochromocytoma cell line PC-12 with CS glycans as a homogeneous substrate, laminin and various CS (CS-A, CS-C, CS-D, CS-E) were coated onto 12 mm glass-bottom dishes. 10 h after NGF administration, the neurite length of PC-12 cells and the number of collapsed growth cones present at the neurite tip site were calculated.

Figure 8 shows the effects of various glycosaminoglycans (CS-A to E) on neurite outgrowth and growth cones of PC-12 cells. Axonal outgrowth was significantly inhibited in the presence of CS-E.

### 5-2. Lactoferrin N-lobe regulates neurite outgrowth by CS-E

The same moles of hLf N-lobe were added to a mixture of laminin and CS-E and the neurite length of PC-12 cells and the number of collapsed growth cones at the neurite tip after 10 hours of addition were determined.

Figure 9 shows that the inhibitory effect on outgrowth of the neurite of PC-12 cells by chondroitin sulfate E is attenuated by the administration of human lactoferrin N-lobe, which promotes neurite outgrowth.

### Example 6: Evaluation of the Binding of hLF, hLF N-lobe, and hLF Derivatives to Chondroitin Sulfate E (CS-E).

CS-E fixation plates were prepared by adding 600 nM biotinylated CS-E (PG Research) to neutral avidin-coated strip-well plates (Thermo Scientific) and allowing them to react for 2 hours at room temperature. After washing each well of the CS-E fixation plate with PBS(-), blocking was performed by shaking for 1 hour in Blocking One (Nacalitesque). Then 1.5 µM hLF, hLF N-lobe, and hLF derivatives (hLF-CH2-CH3, hLF-HSA, and hLF-G-CSF) were added and allowed to react for 4 hours. The primary antibody reaction was performed with HRP-conjugated anti-hLF antibodies (Bethyl Laboratories) diluted 1000-fold in Blocking/Sample Dilution Buffer (Chondrex) for 1 hour at room temperature. After the reaction, the reaction was washed with PBS(-) and then the chromogenic substrate solution TMB (Nacalitesque) was added and allowed to react at room temperature. The reaction was stopped with 1N HCl and the absorbance was measured at 450 nm with a microplate reader. Competitive experiments were conducted with hLF, hLF N-lobe, and hLF derivatives (hLF-CH2-CH3, hLF-HSA, hLF-G-CSF), as well as 3 µM CS-C, CS-E, HP, and HSA, and reacted for 4 hours.

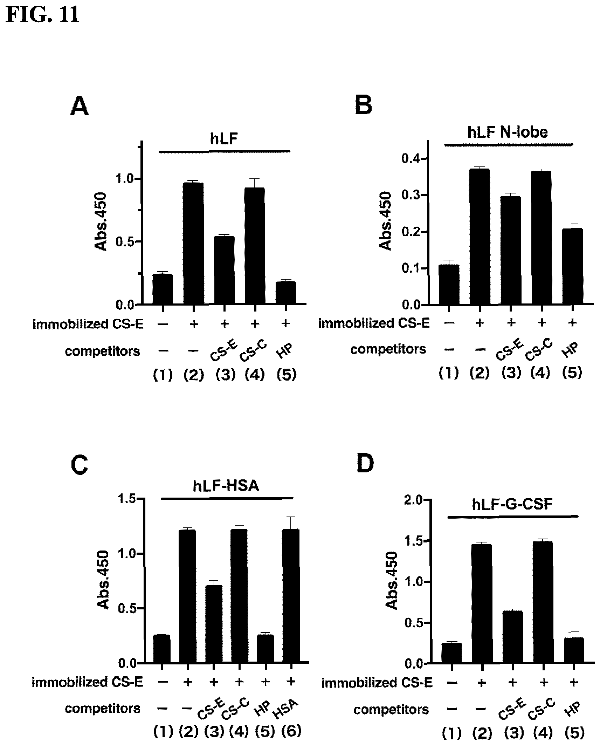

Figure 11A shows the results of binding of hLF to sGAG (CS-E, CS-C, HP). FIG. 11B shows the results of binding of hLF N-lobe to sGAG (CS-E, CS-C, HP). FIG. 11C shows the results of binding of hLF-CH2-CH3 fusion protein (hLF-CH2-CH3) to sGAG (CS-E, CS-C, HP). Figure 11D shows the results of binding of hLF-HSA fusion protein (hLF-HSA) to sGAG (CS-E, CS-C, HP) or human serum albumin (HSA). Figure 11E shows the results of binding of hLF-granulocyte colony-stimulating factor (G-CSF) fusion protein to sGAG (CS-E, CS-C, HP). hLF, hLF N-lobe, and hLF derivatives (hLF-CH2-CH3, hLF-HSA, hLF-G-CSF) directly bound to CS-E (A, B, C, D, E (1) and (2)). When other sGAGs (CS-E, CS-C, HP) or HSAs were acted on the immobilized CS-E at the same time as hLF, hLF N-lobe, and hLF derivatives (hLF-CH2-CH3, hLF-HSA, hLF-G-CSF), there was competitive inhibition for CS-E and HP (A, B, C, C, D, E (3) and (5)), but not in CS-C and HSA (A, B, C, D, E (4) and C (6)).

### Example 7: Effects on Spinal Cord Injury Model Using Chick Embryo Dorsal Root Ganglion Cells

### 7-1. Effects of hLF, hLF globular domain and hLF derivatives on inhibition of CS-E axon growth

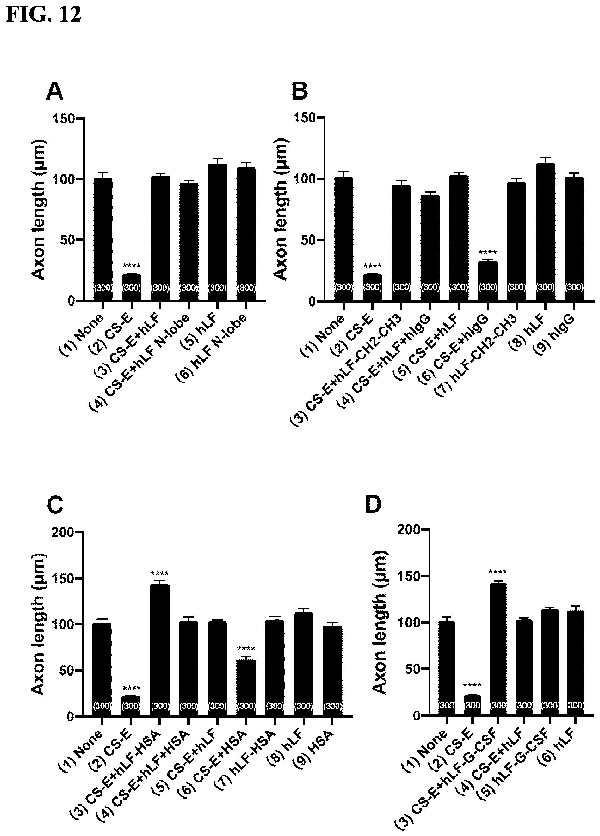

Samples of 10 µg/ml laminin (Gibco), 10 µg/ml CS-E (PG Research), and 138 nM hLF, hLF N-lobe, and a combination of hLF derivatives (hLF-CH2-CH3, hLF-HSA, and hLF-G-CSF) shown in Figure 12, premixed and reacted for 1 hour at 37°C were applied to a 14 mmϕ glass-bottom dish (Matsunami Glass Industries) coated with 100 µg/ml poly-D-lysine (Sigma-Aldrich). Spinal dorsal root ganglion cells were harvested from 8-day-old chick embryos and treated with 0.05% trypsin/EDTA at 37°C for 18 min. DRGs were suspended in Neurobasal medium containing 2% B-27 supplement (Gibco), 1% penicillin-streptomycin solution (Thermo Fisher Scientific), and 20 ng/ml mouse NGF2.5S (Alomone Labs) and incubated at 37°C for 24 h. Axon lengths were measured using WraySpect (Raymer). The longest axon lengths of 300 DRGs were measured and the vertical axis shows the average of the longest axon lengths.

Figure 12A shows the results of the inhibitory effects of hLF and hLF N-lobe on the axon growth inhibitory activity of CS-E. Figure 12B shows the results of the inhibitory effect of hLF-CH2-CH3 on the axon growth inhibitory activity of CS-E. Figure 12C shows the results of the inhibitory effect of hLF-HSA on the axon growth inhibitory activity of CS-E. Figure 12D shows the results of the inhibitory effect of hLF-G-CSF on the axon elongation inhibitory activity of CS-E.

The inhibitory activity of CS-E on axonal outgrowth of DRGs (A, B, C, and D (1) and (2)) was completely suppressed in the presence of hLF (A (3), B (5), C (5), and D (4)), hLF N-lobe (A (4)), hLF-CH2-CH3 (B (3)), hLF-HSA (C (3)), or hLF-G-CSF (D (3))) and the axonal outgrowth was recovered to the level of length of nerve axons (A, B, C, D (1)), which were not acted on by CS-E, or more. With the addition of hLF and hIgG (B (4)) and hLF and HSA (C (4)) as controls for the fusion proteins (hLF-CH2-CH3 and hLF-HSA), respectively, the axonal outgrowth was also recovered to the level of length of nerve axons (A, B, C (1)) that were not acted on by CS-E. In particular, hLF-HSA (C (3)) and hLF-G-CSF (D (3)) enhanced axon elongation compared to CS-E untreated nerve axons (C, D (1)), and no effect was observed when hLF and HSA were added together (C (4)), indicating effectiveness in preparing these proteins as a fusion protein. Also, for nerve axons (A, B, C, and D (1)) without CS-E action, hLF (A (5), B (8), C (8), and D (6)), hLF N-lobe (A (6)), hLF-CH2-CH3 (B (7)), hLF-HSA (C (7)), hLF-G-CSF (D (5)) did not affect axon elongation.

### 7-2. Effects of hLF and hLF Derivatives on growth cone disruption of CS-E

After application of 100 µg/ml poly-D-lysine, 8-day-old chick embryo spinal cord dorsal root ganglion (DRG) cells were seeded into dishes coated with an additional 10 µg/ml laminin and then subjected to a 2% B-27 supplement (Gibco), 1% penicillin-streptomycin solution (Thermo Fisher Scientific) and 20 ng/ml mouse NGF2.5S (Promega) in Neurobasal medium (Thermo Fisher Scientific) culture medium for 24 hours. 10 µg/ml CS-E or 10 µg/ml CS-E coexisted with 138 nM hLF, hLF N-lobe, and hLF derivatives (hLF-CH2-CH3, hLF-HSA, and hLF-G-CSF) in the culture medium, and the decay rate of the growth cones was calculated after 30 minutes. The vertical axis indicates the decay rate in the total growth cones.

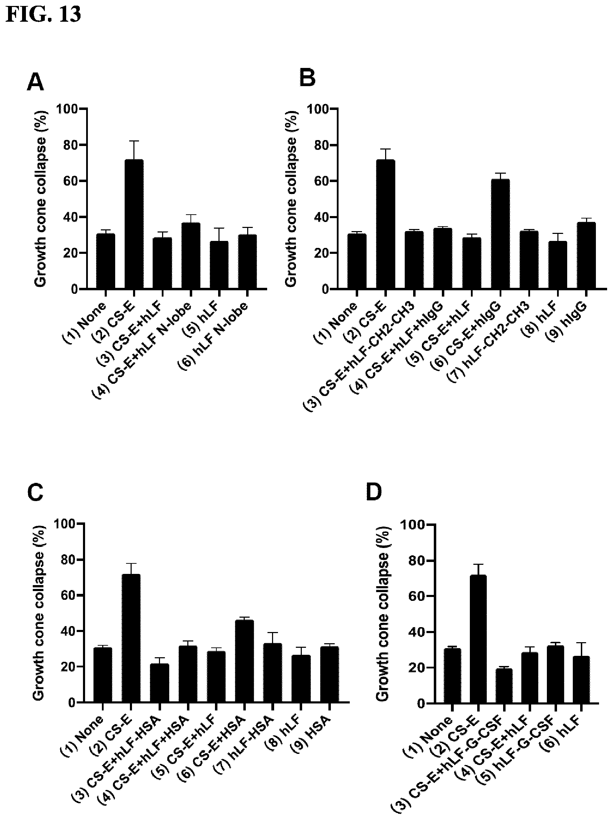

Figure 13A shows the results of the inhibitory effect of hLF and hLF N-lobe on the disruption activity of growth cones by CS-E. FIG. 13B shows the results of the inhibitory action of hLF-CH2-CH3 on the disruption activity of growth cones by CS-E. FIG. 13C shows the result of the inhibitory action of hLF-HSA on the disruption activity of growth cones by CS-E. Figure 13D shows the results of the inhibitory action of hLF-G-CSF on the disruption activity of growth cones by CS-E.

Disruption activity of DRG growth cones by CS-E (A, B, C, and D (1) and (2)) was completely inhibited in the presence of hLF (A (3), B (5), C (5), and D (4)), hLF N-lobe (A (4)), hLF-CH2-CH3 (B (3)), hLF-HSA (C (3)), hLF-G-CSF (D (3))) and recovered to the same extent as the growth cone disruption (A, B, C, and D (1)) in neurons that had not been subjected to CS-E. When hLF and hIgG (B (4)) or hLF and HSA (C (4)) were added together as a control for the fusion proteins (hLF-CH2-CH3 and hLF-HSA), they also recovered to the same extent as the non-CS-E-activated neuronal growth cone disruption (A, B, C (1)). The addition of hLF-HSA (C (3)) and hLF-G-CSF (D (3)) in particular tended to lower the growth cone disruption rate than that of the untreated neurons (C, D (1). In contrast, the effect of preparing them as fusion proteins was shown. Also, neurons (A, B, C, and D (1)) without CS-E (A, B, C, and D (1)) were treated with hLF (A (5), B (8), C (8), and D (6)), hLF N-lobe (A (6)), hLF-CH2-CH3 (B (7)), hLF-HSA (C (7)), hLF-G-CSF (D (5)), respectively, did not affect the growth cone collapse.

### INDUSTRIAL APPLICABILITY

Glycosaminoglycan inhibition/promotion formulations containing lactoferrin or derivatives thereof may be used as a therapeutic agent or in the manufacture of therapeutic agents for the treatment of diseases or conditions related to glycosaminoglycan function.

## Claims

1. An inhibitor or promoter composition of glycosaminoglycans comprising lactoferrin or derivatives thereof.

2. The inhibitor or promoter composition according to claim 1, wherein the lactoferrin is an iron free (Apo) or iron saturated (Holo) lactoferrin.

3. The inhibitor or promoter composition according to claim 1 or 2, wherein the lactoferrin is of human origin.

4. The inhibitor or promoter composition according to claim 1, wherein the lactoferrin is a protein or peptide selected from the group consisting of (a) to (e) below.
(a) a protein consisting of any one amino acid sequence of SEQ ID NOS: 1-6;
(b) a protein comprising N-lobe of lactoferrin;
(c) a protein consisting of the amino acid sequence in which 1 to 70 amino acids are deleted, substituted, inserted, and/or added in any one amino acid sequence of SEQ ID NOS: 1 to 6, and which has activity to inhibit or promote the expression or function of chondroitin sulfate; and
(d) a protein consisting of the amino acid sequence having 90% or more sequence identity to any one amino acid sequence of SEQ ID NOS: 1-6 and having activity that inhibits or promotes the expression or function of chondroitin sulfate; and
(e) a peptide consisting of the amino acid sequence of GRRRRSVQWCA (SEQ ID NO: 7).

5. The inhibitor or promoter composition according to any one of claims 1 to 4, the derivative of the lactoferrin is a hinge deletion human lactoferrin/human IgG Fc fusion protein (hLF-CH2-CH3) (SEQ ID NO: 9), human lactoferrin/human serum albumin fusion protein (hLF (-HSA) (SEQ ID NO: 10) or human lactoferrin-human granulocyte colony-stimulating factor (G-CSF) fusion protein (hLF-G-CSF) (SEQ ID NO: 11).

6. The inhibitor or promoter composition according to any one of claims 1 to 5, wherein the glycosaminoglycan is any one selected from the group consisting of chondroitin sulfate-A (CS-A), chondroitin sulfate-B (CS-B), chondroitin sulfate-C (CS-C), chondroitin sulfate-D (CS-D), chondroitin sulfate-E (CS-E), heparin (HP) and heparan sulfate (HS), and keratan sulfate (KS).

7. The inhibitor or promoter composition according to claim 6, wherein the glycosaminoglycan is chondroitin sulfate-C (CS-C), chondroitin sulfate-D (CS-D), or chondroitin sulfate-E (CS-E).

8. The inhibitor or promoter composition according to claim 6, wherein the glycosaminoglycan is CS-E.

9. A pharmaceutical composition for treating a glycosaminoglycan-related disease or condition, comprising lactoferrin or a derivative thereof.

10. The pharmaceutical composition according to claim 9, wherein the lactoferrin is iron free (Apo) or iron saturated (Holo) lactoferrin, prepared in an amount from 0.001 to 10 g/kg/day.

11. The pharmaceutical composition according to claim 9 or 10, wherein the lactoferrin is of human origin.

12. The pharmaceutical composition according to claim 9, wherein the lactoferrin is a protein or peptide selected from the group consisting of (a) to (e) below:
(a) a protein consisting of any one amino acid sequence of SEQ ID NOS: 1-6;
(b) a protein comprising N-lobe of lactoferrin;
(c) a protein consisting of the amino acid sequence in which 1 to 70 amino acids are deleted, substituted, inserted, and/or added in any one amino acid sequence of SEQ ID NOS: 1 to 6, and which has activity to inhibit or promote the expression or function of glycosaminoglycans; and
(d) a protein consisting of the amino acid sequence having 90% or more sequence identity to any one amino acid sequence of SEQ ID NOS: 1-6 and having activity that inhibits or promotes the expression or function of chondroitin sulfate; and
(e) a peptide consisting of the amino acid sequence of GRRRRSVQWCA (SEQ ID NO: 7).

13. The pharmaceutical composition according to any one of claims 9 to 12, wherein the derivative of the lactoferrin is a hinge deletion human lactoferrin/human IgG Fc fusion protein (hLF-CH2-CH3) (SEQ ID NO: 9), human lactoferrin/human serum albumin fusion protein (hLF-HSA) (SEQ ID NO: 10) or human lactoferrin-human granulocyte colony-stimulating factor (G-CSF) fusion protein (hLF-G-CSF) (SEQ ID NO: 11).

14. The pharmaceutical composition according to any one of claims 9 to 13, wherein the glycosaminoglycan is chondroitin sulfate-C (CS-C), chondroitin sulfate-D (CS-D), or chondroitin sulfate-E (CS-E).

15. The pharmaceutical composition according to any one of claims 9 to 14, for use in combination with chondroitin sulfate-degrading enzymes.

16. The pharmaceutical composition according to any one of claims 9 to 15, wherein the disease is any one selected from the group consisting of spinal cord injury, cancer metastasis, cancer, diabetes, inflammation, atherosclerosis, fibrosis, acute respiratory disease, and Alzheimer's disease.

17. The pharmaceutical composition according to any one of claims 9 to 16, which is in the form of a food product.

18. The pharmaceutical composition according to any one of the claims 9 to 16, which is in the form of an injection.

19. The pharmaceutical composition according to any one of claims 9 to 16, wherein the pharmaceutical composition is for an oral administration.


SEQUENCE LISTING

<110>  NRL Pharma, Inc.

<120>  Glycosaminoglycan Inhibitor and Augmentor

<130>  G2086WO

<150>  JP 2018-121377
<151>  2018-06-26

<160>  11

<170>  PatentIn version 3.5

<210>  1
<211>  711
<212>  PRT
<213>  Homo sapiens

<400>  1

<210>  2
<211>  708
<212>  PRT
<213>  Bos taurus

<400>  2

Met Lys Leu Phe Val Pro Ala Leu Leu Ser Leu Gly Ala Leu Gly Leu
1               5                   10                  15

Cys Leu Ala Ala Pro Arg Lys Asn Val Arg Trp Cys Thr Ile Ser Gln
            20                  25                  30

Pro Glu Trp Phe Lys Cys Arg Arg Trp Gln Trp Arg Met Lys Lys Leu
        35                  40                  45

Gly Ala Pro Ser Ile Thr Cys Val Arg Arg Ala Phe Ala Leu Glu Cys
    50                  55                  60

Ile Arg Ala Ile Ala Glu Lys Lys Ala Asp Ala Val Thr Leu Asp Gly
65                  70                  75                  80

Gly Met Val Phe Glu Ala Gly Arg Asp Pro Tyr Lys Leu Arg Pro Val
                85                  90                  95

Ala Ala Glu Ile Tyr Gly Thr Lys Glu Ser Pro Gln Thr His Tyr Tyr
            100                 105                 110

Ala Val Ala Val Val Lys Lys Gly Ser Asn Phe Gln Leu Asp Gln Leu
        115                 120                 125

Gln Gly Arg Lys Ser Cys His Thr Gly Leu Gly Arg Ser Ala Gly Trp
    130                 135                 140

Ile Ile Pro Met Gly Ile Leu Arg Pro Tyr Leu Ser Trp Thr Glu Ser
145                 150                 155                 160

Leu Glu Pro Leu Gln Gly Ala Val Ala Lys Phe Phe Ser Ala Ser Cys
                165                 170                 175

Val Pro Cys Ile Asp Arg Gln Ala Tyr Pro Asn Leu Cys Gln Leu Cys
            180                 185                 190

Lys Gly Glu Gly Glu Asn Gln Cys Ala Cys Ser Ser Arg Glu Pro Tyr
        195                 200                 205

Phe Gly Tyr Ser Gly Ala Phe Lys Cys Leu Gln Asp Gly Ala Gly Asp
    210                 215                 220

Val Ala Phe Val Lys Glu Thr Thr Val Phe Glu Asn Leu Pro Glu Lys
225                 230                 235                 240

Ala Asp Arg Asp Gln Tyr Glu Leu Leu Cys Leu Asn Asn Ser Arg Ala
                245                 250                 255

Pro Val Asp Ala Phe Lys Glu Cys His Leu Ala Gln Val Pro Ser His
            260                 265                 270

Ala Val Val Ala Arg Ser Val Asp Gly Lys Glu Asp Leu Ile Trp Lys
        275                 280                 285

Leu Leu Ser Lys Ala Gln Glu Lys Phe Gly Lys Asn Lys Ser Arg Ser
    290                 295                 300

Phe Gln Leu Phe Gly Ser Pro Pro Gly Gln Arg Asp Leu Leu Phe Lys
305                 310                 315                 320

Asp Ser Ala Leu Gly Phe Leu Arg Ile Pro Ser Lys Val Asp Ser Ala
                325                 330                 335

Leu Tyr Leu Gly Ser Arg Tyr Leu Thr Thr Leu Lys Asn Leu Arg Glu
            340                 345                 350

Thr Ala Glu Glu Val Lys Ala Arg Tyr Thr Arg Val Val Trp Cys Ala
        355                 360                 365

Val Gly Pro Glu Glu Gln Lys Lys Cys Gln Gln Trp Ser Gln Gln Ser
    370                 375                 380

Gly Gln Asn Val Thr Cys Ala Thr Ala Ser Thr Thr Asp Asp Cys Ile
385                 390                 395                 400

Val Leu Val Leu Lys Gly Glu Ala Asp Ala Leu Asn Leu Asp Gly Gly
                405                 410                 415

Tyr Ile Tyr Thr Ala Gly Lys Cys Gly Leu Val Pro Val Leu Ala Glu
            420                 425                 430

Asn Arg Lys Ser Ser Lys His Ser Ser Leu Asp Cys Val Leu Arg Pro
        435                 440                 445

Thr Glu Gly Tyr Leu Ala Val Ala Val Val Lys Lys Ala Asn Glu Gly
    450                 455                 460

Leu Thr Trp Asn Ser Leu Lys Asp Lys Lys Ser Cys His Thr Ala Val
465                 470                 475                 480

Asp Arg Thr Ala Gly Trp Asn Ile Pro Met Gly Leu Ile Val Asn Gln
                485                 490                 495

Thr Gly Ser Cys Ala Phe Asp Glu Phe Phe Ser Gln Ser Cys Ala Pro
            500                 505                 510

Gly Ala Asp Pro Lys Ser Arg Leu Cys Ala Leu Cys Ala Gly Asp Asp
        515                 520                 525

Gln Gly Leu Asp Lys Cys Val Pro Asn Ser Lys Glu Lys Tyr Tyr Gly
    530                 535                 540

Tyr Thr Gly Ala Phe Arg Cys Leu Ala Glu Asp Val Gly Asp Val Ala
545                 550                 555                 560

Phe Val Lys Asn Asp Thr Val Trp Glu Asn Thr Asn Gly Glu Ser Thr
                565                 570                 575

Ala Asp Trp Ala Lys Asn Leu Asn Arg Glu Asp Phe Arg Leu Leu Cys
            580                 585                 590

Leu Asp Gly Thr Arg Lys Pro Val Thr Glu Ala Gln Ser Cys His Leu
        595                 600                 605

Ala Val Ala Pro Asn His Ala Val Val Ser Arg Ser Asp Arg Ala Ala
    610                 615                 620

His Val Lys Gln Val Leu Leu His Gln Gln Ala Leu Phe Gly Lys Asn
625                 630                 635                 640

Gly Lys Asn Cys Pro Asp Lys Phe Cys Leu Phe Lys Ser Glu Thr Lys
                645                 650                 655

Asn Leu Leu Phe Asn Asp Asn Thr Glu Cys Leu Ala Lys Leu Gly Gly
            660                 665                 670

Arg Pro Thr Tyr Glu Glu Tyr Leu Gly Thr Glu Tyr Val Thr Ala Ile

675                 680                 685

Ala Asn Leu Lys Lys Cys Ser Thr Ser Pro Leu Leu Glu Ala Cys Ala
    690                 695                 700

Phe Leu Thr Arg
705

<210>  3
<211>  708
<212>  PRT
<213>  Ovis aries

<400>  3

Met Lys Leu Phe Val Pro Ala Leu Leu Ser Leu Gly Ala Leu Gly Leu
1               5                   10                  15

Cys Leu Ala Ala Pro Arg Lys Asn Val Arg Trp Cys Ala Ile Ser Pro
            20                  25                  30

Pro Glu Gly Ser Lys Cys Tyr Gln Trp Gln Arg Arg Met Arg Lys Leu
        35                  40                  45

Gly Ala Pro Ser Ile Thr Cys Val Arg Arg Thr Ser Ala Leu Glu Cys
    50                  55                  60

Ile Arg Ala Ile Ala Gly Lys Lys Ala Asp Ala Val Thr Leu Asp Ser
65                  70                  75                  80

Gly Met Val Phe Glu Ala Gly Leu Asp Pro Tyr Lys Leu Arg Pro Val
                85                  90                  95

Ala Ala Glu Ile Tyr Gly Thr Glu Lys Ser Pro Gln Thr His Tyr Tyr
            100                 105                 110

Ala Val Ala Val Val Lys Lys Gly Ser Asn Phe Gln Leu Asp Gln Leu
        115                 120                 125

Gln Gly Gln Lys Ser Cys His Met Gly Leu Gly Arg Ser Ala Gly Trp
    130                 135                 140

Asn Ile Pro Met Gly Ile Leu Arg Pro Phe Leu Ser Trp Thr Glu Ser
145                 150                 155                 160

Ala Glu Pro Leu Gln Gly Ala Val Ala Arg Phe Phe Ser Ala Ser Cys
                165                 170                 175

Val Pro Cys Val Asp Gly Lys Ala Tyr Pro Asn Leu Cys Gln Leu Cys

            180                 185                 190

Lys Gly Val Gly Glu Asn Lys Cys Ala Cys Ser Ser Gln Glu Pro Tyr
        195                 200                 205

Phe Gly Tyr Ser Gly Ala Phe Lys Cys Leu Gln Asp Gly Ala Gly Asp
    210                 215                 220

Val Ala Phe Val Lys Glu Thr Thr Val Phe Glu Asn Leu Pro Glu Lys
225                 230                 235                 240

Ala Asp Arg Asp Gln Tyr Glu Leu Leu Cys Leu Asn Asn Thr Arg Ala
                245                 250                 255

Pro Val Asp Ala Phe Lys Glu Cys His Leu Ala Gln Val Pro Ser His
            260                 265                 270

Ala Val Val Ala Arg Ser Val Asp Gly Lys Glu Asn Leu Ile Trp Glu
        275                 280                 285

Leu Leu Arg Lys Ala Gln Glu Lys Phe Gly Lys Asn Lys Ser Gln Arg
    290                 295                 300

Phe Gln Leu Phe Gly Ser Pro Gln Gly Gln Lys Asp Leu Leu Phe Lys
305                 310                 315                 320

Asp Ser Ala Leu Gly Phe Val Arg Ile Pro Ser Lys Val Asp Ser Ala
                325                 330                 335

Leu Tyr Leu Gly Ser Arg Tyr Leu Thr Ala Leu Lys Asn Leu Arg Glu
            340                 345                 350

Thr Ala Glu Glu Val Lys Ala Arg Cys Thr Arg Val Val Trp Cys Ala
        355                 360                 365

Val Gly Pro Glu Glu His Ser Lys Cys Gln Gln Trp Ser Glu Gln Ser
    370                 375                 380

Gly Gln Asn Val Thr Cys Ala Met Ala Ser Thr Thr Asp Asp Cys Ile
385                 390                 395                 400

Ala Leu Val Leu Lys Gly Glu Ala Asp Ala Leu Ser Leu Asp Gly Gly
                405                 410                 415

Tyr Ile Tyr Thr Ala Gly Lys Cys Gly Leu Val Pro Val Met Ala Glu
            420                 425                 430

Asn Arg Glu Ser Ser Lys Tyr Ser Ser Leu Asp Cys Val Leu Arg Pro
        435                 440                 445

Thr Glu Gly Tyr Leu Ala Val Ala Val Val Lys Lys Ala Asn Glu Gly
    450                 455                 460

Leu Thr Trp Asn Ser Leu Lys Gly Lys Lys Ser Cys His Thr Ala Val
465                 470                 475                 480

Asp Arg Thr Ala Gly Trp Asn Ile Pro Met Gly Leu Ile Ala Asn Gln
                485                 490                 495

Thr Gly Ser Cys Ala Phe Asp Glu Phe Phe Ser Gln Ser Cys Ala Pro
            500                 505                 510

Gly Ala Asp Pro Lys Ser Ser Leu Cys Ala Leu Cys Ala Gly Asp Asp
        515                 520                 525

Gln Gly Leu Asn Lys Cys Val Pro Asn Ser Lys Glu Lys Tyr Tyr Gly
    530                 535                 540

Tyr Thr Gly Ala Phe Arg Cys Leu Ala Glu Asp Val Gly Asp Val Ala
545                 550                 555                 560

Phe Val Lys Asn Asp Thr Val Trp Glu Asn Thr Asn Gly Glu Ser Ser
                565                 570                 575

Ala Asp Trp Ala Lys Asn Leu Asn Arg Glu Asp Phe Arg Leu Leu Cys
            580                 585                 590

Leu Asp Gly Thr Thr Lys Pro Val Thr Glu Ala Gln Ser Cys Tyr Leu
        595                 600                 605

Ala Val Ala Pro Asn His Ala Val Val Ser Arg Ser Asp Arg Ala Ala
    610                 615                 620

His Val Glu Gln Val Leu Leu His Gln Gln Ala Leu Phe Gly Lys Asn
625                 630                 635                 640

Gly Lys Asn Cys Pro Asp Gln Phe Cys Leu Phe Lys Ser Glu Thr Lys
                645                 650                 655

Asn Leu Leu Phe Asn Asp Asn Thr Glu Cys Leu Ala Lys Leu Gly Gly
            660                 665                 670

Arg Pro Thr Tyr Glu Lys Tyr Leu Gly Thr Glu Tyr Val Thr Ala Ile
        675                 680                 685

Ala Asn Leu Lys Lys Cys Ser Thr Ser Pro Leu Leu Glu Ala Cys Ala
    690                 695                 700

Phe Leu Thr Arg
705

<210>  4
<211>  708
<212>  PRT
<213>  Capra hircus

<400>  4

Met Lys Leu Phe Val Pro Ala Leu Leu Ser Leu Gly Ala Leu Gly Leu
1               5                   10                  15

Cys Leu Ala Ala Pro Arg Lys Asn Val Arg Trp Cys Ala Ile Ser Leu
            20                  25                  30

Pro Glu Trp Ser Lys Cys Tyr Gln Trp Gln Arg Arg Met Arg Lys Leu
        35                  40                  45

Gly Ala Pro Ser Ile Thr Cys Ile Arg Arg Thr Ser Ala Leu Glu Cys
    50                  55                  60

Ile Arg Ala Ile Ala Gly Lys Asn Ala Asp Ala Val Thr Leu Asp Ser
65                  70                  75                  80

Gly Met Val Phe Glu Ala Gly Leu Asp Pro Tyr Lys Leu Arg Pro Val
                85                  90                  95

Ala Ala Glu Ile Tyr Gly Thr Glu Lys Ser Pro Gln Thr His Tyr Tyr
            100                 105                 110

Ala Val Ala Val Val Lys Lys Gly Ser Asn Phe Gln Leu Asp Gln Leu
        115                 120                 125

Gln Gly Gln Lys Ser Cys His Met Gly Leu Gly Arg Ser Ala Gly Trp
    130                 135                 140

Asn Ile Pro Val Gly Ile Leu Arg Pro Phe Leu Ser Trp Thr Glu Ser
145                 150                 155                 160

Ala Glu Pro Leu Gln Gly Ala Val Ala Arg Phe Phe Ser Ala Ser Cys
                165                 170                 175

Val Pro Cys Val Asp Gly Lys Ala Tyr Pro Asn Leu Cys Gln Leu Cys
            180                 185                 190

Lys Gly Val Gly Glu Asn Lys Cys Ala Cys Ser Ser Gln Glu Pro Tyr
        195                 200                 205

Phe Gly Tyr Ser Gly Ala Phe Lys Cys Leu Gln Asp Gly Ala Gly Asp
    210                 215                 220

Val Ala Phe Val Lys Glu Thr Thr Val Phe Glu Asn Leu Pro Glu Lys
225                 230                 235                 240

Ala Asp Arg Asp Gln Tyr Glu Leu Leu Cys Leu Asn Asn Thr Arg Ala
                245                 250                 255

Pro Val Asp Ala Phe Lys Glu Cys His Leu Ala Gln Val Pro Ser His
            260                 265                 270

Ala Val Val Ala Arg Ser Val Asp Gly Lys Glu Asn Leu Ile Trp Glu
        275                 280                 285

Leu Leu Arg Lys Ala Gln Glu Lys Phe Gly Lys Asn Lys Ser Gln Ser
    290                 295                 300

Phe Gln Leu Phe Gly Ser Pro Glu Gly Arg Arg Asp Leu Leu Phe Lys
305                 310                 315                 320

Asp Ser Ala Leu Gly Phe Val Arg Ile Pro Ser Lys Val Asp Ser Ala
                325                 330                 335

Leu Tyr Leu Gly Ser Arg Tyr Leu Thr Ala Leu Lys Asn Leu Arg Glu
            340                 345                 350

Thr Ala Glu Glu Leu Lys Ala Arg Cys Thr Arg Val Val Trp Cys Ala
        355                 360                 365

Val Gly Pro Glu Glu Gln Ser Lys Cys Gln Gln Trp Ser Glu Gln Ser
    370                 375                 380

Gly Gln Asn Val Thr Cys Ala Thr Ala Ser Thr Thr Asp Asp Cys Ile
385                 390                 395                 400

Ala Leu Val Leu Lys Gly Glu Ala Asp Ala Leu Ser Leu Asp Gly Gly
                405                 410                 415

Tyr Ile Tyr Thr Ala Gly Lys Cys Gly Leu Val Pro Val Met Ala Glu
            420                 425                 430

Asn Arg Lys Ser Ser Lys Tyr Ser Ser Leu Asp Cys Val Leu Arg Pro
        435                 440                 445

Thr Glu Gly Tyr Leu Ala Val Ala Val Val Lys Lys Ala Asn Glu Gly
    450                 455                 460

Leu Thr Trp Asn Ser Leu Lys Gly Lys Lys Ser Cys His Thr Ala Val
465                 470                 475                 480

Asp Arg Thr Ala Gly Trp Asn Ile Pro Met Gly Leu Ile Ala Asn Gln
                485                 490                 495

Thr Gly Ser Cys Ala Phe Asp Glu Phe Phe Ser Gln Ser Cys Ala Pro
            500                 505                 510

Gly Ala Asp Pro Lys Ser Ser Leu Cys Ala Leu Cys Ala Gly Asp Asp
        515                 520                 525

Gln Gly Leu Asp Lys Cys Val Pro Asn Ser Lys Glu Lys Tyr Tyr Gly
    530                 535                 540

Tyr Thr Gly Ala Phe Arg Cys Leu Ala Glu Asp Val Gly Asp Val Ala
545                 550                 555                 560

Phe Val Lys Asn Asp Thr Val Trp Glu Asn Thr Asn Gly Glu Ser Ser
                565                 570                 575

Ala Asp Trp Ala Lys Asn Leu Asn Arg Glu Asp Phe Arg Leu Leu Cys
            580                 585                 590

Leu Asp Gly Thr Thr Lys Pro Val Thr Glu Ala Gln Ser Cys Tyr Leu
        595                 600                 605

Ala Val Ala Pro Asn His Ala Val Val Ser Arg Ser Asp Arg Ala Ala
    610                 615                 620

His Val Glu Gln Val Leu Leu His Gln Gln Ala Leu Phe Gly Lys Asn
625                 630                 635                 640

Gly Lys Asn Cys Pro Asp Gln Phe Cys Leu Phe Lys Ser Glu Thr Lys
                645                 650                 655

Asn Leu Leu Phe Asn Asp Asn Thr Glu Cys Leu Ala Lys Leu Gly Gly
            660                 665                 670

Arg Pro Thr Tyr Glu Lys Tyr Leu Gly Thr Glu Tyr Val Thr Ala Ile
        675                 680                 685

Ala Asn Leu Lys Lys Cys Ser Thr Ser Pro Leu Leu Glu Ala Cys Ala

690                 695                 700

Phe Leu Thr Arg
705

<210>  5
<211>  708
<212>  PRT
<213>  Equus caballus

<400>  5

Met Lys Leu Leu Phe Pro Val Leu Leu Ser Leu Gly Ala Leu Gly Leu
1               5                   10                  15

Cys Leu Ala Ala Pro Arg Lys Ser Val Arg Trp Cys Thr Ile Ser Pro
            20                  25                  30

Ala Glu Ala Ala Lys Cys Ala Lys Phe Gln Arg Asn Met Lys Lys Val
        35                  40                  45

Arg Gly Pro Ser Val Ser Cys Ile Arg Lys Thr Ser Ser Phe Glu Cys
    50                  55                  60

Ile Gln Ala Ile Ala Ala Asn Lys Ala Asp Ala Val Thr Leu Asp Gly
65                  70                  75                  80

Gly Leu Val Tyr Glu Ala Gly Leu His Pro Tyr Lys Leu Arg Pro Val
                85                  90                  95

Ala Ala Glu Val Tyr Gln Thr Arg Gly Lys Pro Gln Thr Arg Tyr Tyr
            100                 105                 110

Ala Val Ala Val Val Lys Lys Gly Ser Gly Phe Gln Leu Asn Gln Leu
        115                 120                 125

Gln Gly Val Lys Ser Cys His Thr Gly Leu Gly Arg Ser Ala Gly Trp
    130                 135                 140

Asn Ile Pro Ile Gly Thr Leu Arg Pro Tyr Leu Asn Trp Thr Gly Pro
145                 150                 155                 160

Pro Glu Pro Leu Gln Lys Ala Val Ala Asn Phe Phe Ser Ala Ser Cys
                165                 170                 175

Val Pro Cys Ala Asp Gly Lys Gln Tyr Pro Asn Leu Cys Arg Leu Cys
            180                 185                 190

Ala Gly Thr Glu Ala Asp Lys Cys Ala Cys Ser Ser Gln Glu Pro Tyr

195                 200                 205

Phe Gly Tyr Ser Gly Ala Phe Lys Cys Leu Glu Asn Gly Ala Gly Asp
    210                 215                 220

Val Ala Phe Val Lys Asp Ser Thr Val Phe Glu Asn Leu Pro Asp Glu
225                 230                 235                 240

Ala Asp Arg Asp Lys Tyr Glu Leu Leu Cys Pro Asp Asn Thr Arg Lys
                245                 250                 255

Pro Val Asp Ala Phe Lys Glu Cys His Leu Ala Arg Val Pro Ser His
            260                 265                 270

Ala Val Val Ala Arg Ser Val Asp Gly Arg Glu Asp Leu Ile Trp Arg
        275                 280                 285

Leu Leu His Arg Ala Gln Glu Glu Phe Gly Arg Asn Lys Ser Ser Ala
    290                 295                 300

Phe Gln Leu Phe Lys Ser Thr Pro Glu Asn Lys Asp Leu Leu Phe Lys
305                 310                 315                 320

Asp Ser Ala Leu Gly Phe Val Arg Ile Pro Ser Gln Ile Asp Ser Gly
                325                 330                 335

Leu Tyr Leu Gly Ala Asn Tyr Leu Thr Ala Thr Gln Asn Leu Arg Glu
            340                 345                 350

Thr Ala Ala Glu Val Ala Ala Arg Arg Glu Arg Val Val Trp Cys Ala
        355                 360                 365

Val Gly Pro Glu Glu Glu Arg Lys Cys Lys Gln Trp Ser Asp Val Ser
    370                 375                 380

Asn Arg Lys Val Ala Cys Ala Ser Ala Ser Thr Thr Glu Glu Cys Ile
385                 390                 395                 400

Ala Leu Val Leu Lys Gly Glu Ala Asp Ala Leu Asn Leu Asp Gly Gly
                405                 410                 415

Phe Ile Tyr Val Ala Gly Lys Cys Gly Leu Val Pro Val Leu Ala Glu
            420                 425                 430

Asn Gln Lys Ser Gln Asn Ser Asn Ala Pro Asp Cys Val His Arg Pro
        435                 440                 445

Pro Glu Gly Tyr Leu Ala Val Ala Val Val Arg Lys Ser Asp Ala Asp
    450                 455                 460

Leu Thr Trp Asn Ser Leu Ser Gly Lys Lys Ser Cys His Thr Gly Val
465                 470                 475                 480

Gly Arg Thr Ala Gly Trp Asn Ile Pro Met Gly Leu Leu Phe Asn Gln
                485                 490                 495

Thr Gly Ser Cys Lys Phe Asp Lys Phe Phe Ser Gln Ser Cys Ala Pro
            500                 505                 510

Gly Ala Asp Pro Gln Ser Ser Leu Cys Ala Leu Cys Val Gly Asn Asn
        515                 520                 525

Glu Asn Glu Asn Lys Cys Met Pro Asn Ser Glu Glu Arg Tyr Tyr Gly
    530                 535                 540

Tyr Thr Gly Ala Phe Arg Cys Leu Ala Glu Lys Ala Gly Asp Val Ala
545                 550                 555                 560

Phe Val Lys Asp Val Thr Val Leu Gln Asn Thr Asp Gly Lys Asn Ser
                565                 570                 575

Glu Pro Trp Ala Lys Asp Leu Lys Gln Glu Asp Phe Glu Leu Leu Cys
            580                 585                 590

Leu Asp Gly Thr Arg Lys Pro Val Ala Glu Ala Glu Ser Cys His Leu
        595                 600                 605

Ala Arg Ala Pro Asn His Ala Val Val Ser Gln Ser Asp Arg Ala Gln
    610                 615                 620

His Leu Lys Lys Val Leu Phe Leu Gln Gln Asp Gln Phe Gly Gly Asn
625                 630                 635                 640

Gly Pro Asp Cys Pro Gly Lys Phe Cys Leu Phe Lys Ser Glu Thr Lys
                645                 650                 655

Asn Leu Leu Phe Asn Asp Asn Thr Glu Cys Leu Ala Glu Leu Gln Gly
            660                 665                 670

Lys Thr Thr Tyr Glu Gln Tyr Leu Gly Ser Glu Tyr Val Thr Ser Ile
        675                 680                 685

Thr Asn Leu Arg Arg Cys Ser Ser Ser Pro Leu Leu Glu Ala Cys Ala
    690                 695                 700

Phe Leu Arg Ala
705

<210> 6
<211> 708
<212> PRT
<213> Camelus dromedarius

<400> 6

Met Lys Leu Phe Phe Pro Ala Leu Leu Ser Leu Gly Ala Leu Gly Leu
1               5                   10                  15

Cys Leu Ala Ala Ser Lys Lys Ser Val Arg Trp Cys Thr Thr Ser Pro
            20                  25                  30

Ala Glu Ser Ser Lys Cys Ala Gln Trp Gln Arg Arg Met Lys Lys Val
        35                  40                  45

Arg Gly Pro Ser Val Thr Cys Val Lys Lys Thr Ser Arg Phe Glu Cys
    50                  55                  60

Ile Gln Ala Ile Ser Thr Glu Lys Ala Asp Ala Val Thr Leu Asp Gly
65                  70                  75                  80

Gly Leu Val Tyr Asp Ala Gly Leu Asp Pro Tyr Lys Leu Arg Pro Ile
                85                  90                  95

Ala Ala Glu Val Tyr Gly Thr Glu Asn Asn Pro Gln Thr His Tyr Tyr
            100                 105                 110

Ala Val Ala Ile Ala Lys Lys Gly Thr Asn Phe Gln Leu Asn Gln Leu
        115                 120                 125

Gln Gly Leu Lys Ser Cys His Thr Gly Leu Gly Arg Ser Ala Gly Trp
    130                 135                 140

Asn Ile Pro Met Gly Leu Leu Arg Pro Phe Leu Asp Trp Thr Gly Pro
145                 150                 155                 160

Pro Glu Pro Leu Gln Lys Ala Val Ala Lys Phe Phe Ser Ala Ser Cys
                165                 170                 175

Val Pro Cys Val Asp Gly Lys Glu Tyr Pro Asn Leu Cys Gln Leu Cys
            180                 185                 190

Ala Gly Thr Gly Glu Asn Lys Cys Ala Cys Ser Ser Gln Glu Pro Tyr
        195                 200                 205

Phe Gly Tyr Ser Gly Ala Phe Lys Cys Leu Gln Asp Gly Ala Gly Asp
    210                 215                 220

Val Ala Phe Val Lys Asp Ser Thr Val Phe Glu Ser Leu Pro Ala Lys
225                 230                 235                 240

Ala Asp Arg Asp Gln Tyr Glu Leu Leu Cys Pro Asn Asn Thr Arg Lys
                245                 250                 255

Pro Val Asp Ala Ser Gln Glu Cys His Leu Ala Arg Val Pro Ser His
            260                 265                 270

Ala Val Val Ala Arg Ser Val Asn Gly Lys Glu Asp Leu Ile Trp Lys
        275                 280                 285

Leu Leu Val Lys Ala Gln Glu Lys Phe Gly Arg Gly Lys Pro Ser Ala
    290                 295                 300

Phe Gln Leu Phe Gly Ser Pro Ala Gly Gln Lys Asp Leu Leu Phe Lys
305                 310                 315                 320

Asp Ser Ala Leu Gly Leu Leu Arg Ile Pro Ser Lys Ile Asp Ser Gly
                325                 330                 335

Leu Tyr Leu Gly Ser Asn Tyr Ile Thr Ala Ile Arg Gly Leu Arg Glu
            340                 345                 350

Thr Ala Ala Glu Val Glu Leu Arg Arg Ala Gln Val Val Trp Cys Ala
        355                 360                 365

Val Gly Ser Asp Glu Gln Leu Lys Cys Gln Glu Trp Ser Arg Gln Ser
    370                 375                 380

Asn Gln Ser Val Val Cys Ala Thr Ala Ser Thr Thr Glu Asp Cys Ile
385                 390                 395                 400

Ala Leu Val Leu Lys Gly Glu Ala Asp Ala Leu Ser Leu Asp Gly Gly
                405                 410                 415

Tyr Ile Tyr Ile Ala Gly Lys Cys Gly Leu Val Pro Val Leu Ala Glu
            420                 425                 430

Ser Gln Gln Ser Pro Glu Ser Ser Gly Leu Asp Cys Val His Arg Pro
        435                 440                 445

Val Lys Gly Tyr Leu Ala Val Ala Val Val Arg Lys Ala Asn Asp Lys
    450                 455                 460

Ile Thr Trp Asn Ser Leu Arg Gly Lys Lys Ser Cys His Thr Ala Val
465                 470                 475                 480

Asp Arg Thr Ala Gly Trp Asn Ile Pro Met Gly Pro Leu Phe Lys Asn
                485                 490                 495

Thr Asp Ser Cys Arg Phe Asp Glu Phe Phe Ser Gln Ser Cys Ala Pro
            500                 505                 510

Gly Ser Asp Pro Arg Ser Lys Leu Cys Ala Leu Cys Ala Gly Asn Glu
        515                 520                 525

Glu Gly Gln Asn Lys Cys Val Pro Asn Ser Ser Glu Arg Tyr Tyr Gly
    530                 535                 540

Tyr Thr Gly Ala Phe Arg Cys Leu Ala Glu Asn Val Gly Asp Val Ala
545                 550                 555                 560

Phe Val Lys Asp Val Thr Val Leu Asp Asn Thr Asp Gly Lys Asn Thr
                565                 570                 575

Glu Gln Trp Ala Lys Asp Leu Lys Leu Gly Asp Phe Glu Leu Leu Cys
            580                 585                 590

Leu Asn Gly Thr Arg Lys Pro Val Thr Glu Ala Glu Ser Cys His Leu
        595                 600                 605

Pro Val Ala Pro Asn His Ala Val Val Ser Arg Ile Asp Lys Val Ala
    610                 615                 620

His Leu Glu Gln Val Leu Leu Arg Gln Gln Ala His Phe Gly Arg Asn
625                 630                 635                 640

Gly Gln Asp Cys Pro Gly Lys Phe Cys Leu Phe Gln Ser Lys Thr Lys
                645                 650                 655

Asn Leu Leu Phe Asn Asp Asn Thr Glu Cys Leu Ala Lys Leu Gln Gly
            660                 665                 670

Lys Thr Thr Tyr Glu Glu Tyr Leu Gly Pro Gln Tyr Val Thr Ala Ile
        675                 680                 685

Ala Lys Leu Arg Arg Cys Ser Thr Ser Pro Leu Leu Glu Ala Cys Ala
    690                 695                 700

Phe Leu Met Arg

705

<210>  7
<211>  11
<212>  PRT
<213>  Artificial Sequence

<220>
<223>  hLfcin1-11 (hLf peptide)

<400>  7

Gly Arg Arg Arg Arg Ser Val Gln Trp Cys Ala
1               5                   10

<210>  8
<211>  351
<212>  PRT
<213>  Homo sapiens

<400>  8

Met Lys Leu Val Phe Leu Val Leu Leu Phe Leu Gly Ala Leu Gly Leu
1               5                   10                  15

Cys Leu Ala Gly Arg Arg Arg Arg Ser Val Gln Trp Cys Ala Val Ser
            20                  25                  30

Gln Pro Glu Ala Thr Lys Cys Phe Gln Trp Gln Arg Asn Met Arg Lys
        35                  40                  45

Val Arg Gly Pro Pro Val Ser Cys Ile Lys Arg Asp Ser Pro Ile Gln
    50                  55                  60

Cys Ile Gln Ala Ile Ala Glu Asn Arg Ala Asp Ala Val Thr Leu Asp
65                  70                  75                  80

Gly Gly Phe Ile Tyr Glu Ala Gly Leu Ala Pro Tyr Lys Leu Arg Pro
                85                  90                  95

Val Ala Ala Glu Val Tyr Gly Thr Glu Arg Gln Pro Arg Thr His Tyr
            100                 105                 110

Tyr Ala Val Ala Val Val Lys Lys Gly Gly Ser Phe Gln Leu Asn Glu
        115                 120                 125

Leu Gln Gly Leu Lys Ser Cys His Thr Gly Leu Arg Arg Thr Ala Gly
    130                 135                 140

Trp Asn Val Pro Ile Gly Thr Leu Arg Pro Phe Leu Asn Trp Thr Gly
145                 150                 155                 160

Pro Pro Glu Pro Ile Glu Ala Ala Val Ala Arg Phe Phe Ser Ala Ser
                165                 170                 175

Cys Val Pro Gly Ala Asp Lys Gly Gln Phe Pro Asn Leu Cys Arg Leu
            180                 185                 190

Cys Ala Gly Thr Gly Glu Asn Lys Cys Ala Phe Ser Ser Gln Glu Pro
        195                 200                 205

Tyr Phe Ser Tyr Ser Gly Ala Phe Lys Cys Leu Arg Asp Gly Ala Gly
    210                 215                 220

Asp Val Ala Phe Ile Arg Glu Ser Thr Val Phe Glu Asp Leu Ser Asp
225                 230                 235                 240

Glu Ala Glu Arg Asp Glu Tyr Glu Leu Leu Cys Pro Asp Asn Thr Arg
                245                 250                 255

Lys Pro Val Asp Lys Phe Lys Asp Cys His Leu Ala Arg Val Pro Ser
            260                 265                 270

His Ala Val Val Ala Arg Ser Val Asn Gly Lys Glu Asp Ala Ile Trp
        275                 280                 285

Asn Leu Leu Arg Gln Ala Gln Glu Lys Phe Gly Lys Asp Lys Ser Pro
    290                 295                 300

Lys Phe Gln Leu Phe Gly Ser Pro Ser Gly Gln Lys Asp Leu Leu Phe
305                 310                 315                 320

Lys Asp Ser Ala Ile Gly Phe Ser Arg Val Pro Pro Arg Ile Asp Ser
                325                 330                 335

Gly Leu Tyr Leu Gly Ser Gly Tyr Phe Thr Ala Ile Gln Asn Leu
            340                 345                 350

<210>  9
<211>  930
<212>  PRT
<213>  Artificial Sequence

<220>
<223>  hLF-CH2-CH3 fusion protein

<400>  9

Met Lys Leu Val Phe Leu Val Leu Leu Phe Leu Gly Ala Leu Gly Leu
1               5                   10                  15

Cys Leu Ala Gly Arg Arg Arg Arg Ser Val Gln Trp Cys Ala Val Ser
            20                  25                  30
Gln Pro Glu Ala Thr Lys Cys Phe Gln Trp Gln Arg Asn Met Arg Lys
        35                  40                  45
Val Arg Gly Pro Pro Val Ser Cys Ile Lys Arg Asp Ser Pro Ile Gln
    50                  55                  60
Cys Ile Gln Ala Ile Ala Glu Asn Arg Ala Asp Ala Val Thr Leu Asp
65                  70                  75                  80
Gly Gly Phe Ile Tyr Glu Ala Gly Leu Ala Pro Tyr Lys Leu Arg Pro
                85                  90                  95
Val Ala Ala Glu Val Tyr Gly Thr Glu Arg Gln Pro Arg Thr His Tyr
            100                 105                 110
Tyr Ala Val Ala Val Val Lys Lys Gly Gly Ser Phe Gln Leu Asn Glu
        115                 120                 125
Leu Gln Gly Leu Lys Ser Cys His Thr Gly Leu Arg Arg Thr Ala Gly
    130                 135                 140
Trp Asn Val Pro Ile Gly Thr Leu Arg Pro Phe Leu Asn Trp Thr Gly
145                 150                 155                 160
Pro Pro Glu Pro Ile Glu Ala Ala Val Ala Arg Phe Phe Ser Ala Ser
                165                 170                 175
Cys Val Pro Gly Ala Asp Lys Gly Gln Phe Pro Asn Leu Cys Arg Leu
            180                 185                 190
Cys Ala Gly Thr Gly Glu Asn Lys Cys Ala Phe Ser Ser Gln Glu Pro
        195                 200                 205
Tyr Phe Ser Tyr Ser Gly Ala Phe Lys Cys Leu Arg Asp Gly Ala Gly
    210                 215                 220
Asp Val Ala Phe Ile Arg Glu Ser Thr Val Phe Glu Asp Leu Ser Asp
225                 230                 235                 240
Glu Ala Glu Arg Asp Glu Tyr Glu Leu Leu Cys Pro Asp Asn Thr Arg
                245                 250                 255
Lys Pro Val Asp Lys Phe Lys Asp Cys His Leu Ala Arg Val Pro Ser
            260                 265                 270

His Ala Val Val Ala Arg Ser Val Asn Gly Lys Glu Asp Ala Ile Trp
        275                 280                 285

Asn Leu Leu Arg Gln Ala Gln Glu Lys Phe Gly Lys Asp Lys Ser Pro
    290                 295                 300

Lys Phe Gln Leu Phe Gly Ser Pro Ser Gly Gln Lys Asp Leu Leu Phe
305                 310                 315                 320

Lys Asp Ser Ala Ile Gly Phe Ser Arg Val Pro Pro Arg Ile Asp Ser
                325                 330                 335

Gly Leu Tyr Leu Gly Ser Gly Tyr Phe Thr Ala Ile Gln Asn Leu Arg
            340                 345                 350

Lys Ser Glu Glu Glu Val Ala Ala Arg Arg Ala Arg Val Val Trp Cys
        355                 360                 365

Ala Val Gly Glu Gln Glu Leu Arg Lys Cys Asn Gln Trp Ser Gly Leu
    370                 375                 380

Ser Glu Gly Ser Val Thr Cys Ser Ser Ala Ser Thr Thr Glu Asp Cys
385                 390                 395                 400

Ile Ala Leu Val Leu Lys Gly Glu Ala Asp Ala Met Ser Leu Asp Gly
                405                 410                 415

Gly Tyr Val Tyr Thr Ala Gly Lys Cys Gly Leu Val Pro Val Leu Ala
            420                 425                 430

Glu Asn Tyr Lys Ser Gln Gln Ser Ser Asp Pro Asp Pro Asn Cys Val
        435                 440                 445

Asp Arg Pro Val Glu Gly Tyr Leu Ala Val Ala Val Val Arg Arg Ser
    450                 455                 460

Asp Thr Ser Leu Thr Trp Asn Ser Val Lys Gly Lys Lys Ser Cys His
465                 470                 475                 480

Thr Ala Val Asp Arg Thr Ala Gly Trp Asn Ile Pro Met Gly Leu Leu
                485                 490                 495

Phe Asn Gln Thr Gly Ser Cys Lys Phe Asp Glu Tyr Phe Ser Gln Ser
            500                 505                 510

Cys Ala Pro Gly Ser Asp Pro Arg Ser Asn Leu Cys Ala Leu Cys Ile
        515                 520                 525

Gly Asp Glu Gln Gly Glu Asn Lys Cys Val Pro Asn Ser Asn Glu Arg
    530                 535                 540

Tyr Tyr Gly Tyr Thr Gly Ala Phe Arg Cys Leu Ala Glu Asn Ala Gly
545                 550                 555                 560

Asp Val Ala Phe Val Lys Asp Val Thr Val Leu Gln Asn Thr Asp Gly
                565                 570                 575

Asn Asn Asn Glu Ala Trp Ala Lys Asp Leu Lys Leu Ala Asp Phe Ala
            580                 585                 590

Leu Leu Cys Leu Asp Gly Lys Arg Lys Pro Val Thr Glu Ala Arg Ser
        595                 600                 605

Cys His Leu Ala Met Ala Pro Asn His Ala Val Val Ser Arg Met Asp
    610                 615                 620

Lys Val Glu Arg Leu Lys Gln Val Leu Leu His Gln Gln Ala Lys Phe
625                 630                 635                 640

Gly Arg Asn Gly Ser Asp Cys Pro Asp Lys Phe Cys Leu Phe Gln Ser
                645                 650                 655

Glu Thr Lys Asn Leu Leu Phe Asn Asp Asn Thr Glu Cys Leu Ala Arg
            660                 665                 670

Leu His Gly Lys Thr Thr Tyr Glu Lys Tyr Leu Gly Pro Gln Tyr Val
        675                 680                 685

Ala Gly Ile Thr Asn Leu Lys Lys Cys Ser Thr Ser Pro Leu Leu Glu
    690                 695                 700

Ala Cys Glu Phe Leu Arg Lys Asp Pro Ala Pro Glu Leu Leu Gly Gly
705                 710                 715                 720

Pro Ser Val Phe Leu Phe Pro Pro Lys Pro Lys Asp Thr Leu Met Ile
                725                 730                 735

Ser Arg Thr Pro Glu Val Thr Cys Val Val Val Asp Val Ser His Glu
            740                 745                 750

Asp Pro Glu Val Lys Phe Asn Trp Tyr Val Asp Gly Val Glu Val His
        755                 760                 765

Asn Ala Lys Thr Lys Pro Arg Glu Glu Gln Tyr Asn Ser Thr Tyr Arg

770                 775                 780

Val Val Ser Val Leu Thr Val Leu His Gln Asp Trp Leu Asn Gly Lys
785                 790                 795                 800

Glu Tyr Lys Cys Lys Val Ser Asn Lys Ala Leu Pro Ala Pro Ile Glu
                805                 810                 815

Lys Thr Ile Ser Lys Ala Lys Gly Gln Pro Arg Glu Pro Gln Val Tyr
            820                 825                 830

Thr Leu Pro Pro Ser Arg Asp Glu Leu Thr Lys Asn Gln Val Ser Leu
        835                 840                 845

Thr Cys Leu Val Lys Gly Phe Tyr Pro Ser Asp Ile Ala Val Glu Trp
    850                 855                 860

Glu Ser Asn Gly Gln Pro Glu Asn Asn Tyr Lys Thr Thr Pro Pro Val
865                 870                 875                 880

Leu Asp Ser Asp Gly Ser Phe Phe Leu Tyr Ser Lys Leu Thr Val Asp
                885                 890                 895

Lys Ser Arg Trp Gln Gln Gly Asn Val Phe Ser Cys Ser Val Met His
            900                 905                 910

Glu Ala Leu His Asn His Tyr Thr Gln Lys Ser Leu Ser Leu Ser Pro
        915                 920                 925

Gly Lys
    930

<210>  10
<211>  1298
<212>  PRT
<213>  Artificial Sequence

<220>
<223>  hLF-HSA fusion protein

<400>  10

Met Lys Leu Val Phe Leu Val Leu Leu Phe Leu Gly Ala Leu Gly Leu
1               5                   10                  15

Cys Leu Ala Gly Arg Arg Arg Arg Ser Val Gln Trp Cys Ala Val Ser
            20                  25                  30

Gln Pro Glu Ala Thr Lys Cys Phe Gln Trp Gln Arg Asn Met Arg Lys
        35                  40                  45

Val Arg Gly Pro Pro Val Ser Cys Ile Lys Arg Asp Ser Pro Ile Gln
    50                  55                  60

Cys Ile Gln Ala Ile Ala Glu Asn Arg Ala Asp Ala Val Thr Leu Asp
65                  70                  75                  80

Gly Gly Phe Ile Tyr Glu Ala Gly Leu Ala Pro Tyr Lys Leu Arg Pro
                85                  90                  95

Val Ala Ala Glu Val Tyr Gly Thr Glu Arg Gln Pro Arg Thr His Tyr
            100                 105                 110

Tyr Ala Val Ala Val Val Lys Lys Gly Gly Ser Phe Gln Leu Asn Glu
        115                 120                 125

Leu Gln Gly Leu Lys Ser Cys His Thr Gly Leu Arg Arg Thr Ala Gly
    130                 135                 140

Trp Asn Val Pro Ile Gly Thr Leu Arg Pro Phe Leu Asn Trp Thr Gly
145                 150                 155                 160

Pro Pro Glu Pro Ile Glu Ala Ala Val Ala Arg Phe Phe Ser Ala Ser
                165                 170                 175

Cys Val Pro Gly Ala Asp Lys Gly Gln Phe Pro Asn Leu Cys Arg Leu
            180                 185                 190

Cys Ala Gly Thr Gly Glu Asn Lys Cys Ala Phe Ser Ser Gln Glu Pro
        195                 200                 205

Tyr Phe Ser Tyr Ser Gly Ala Phe Lys Cys Leu Arg Asp Gly Ala Gly
    210                 215                 220

Asp Val Ala Phe Ile Arg Glu Ser Thr Val Phe Glu Asp Leu Ser Asp
225                 230                 235                 240

Glu Ala Glu Arg Asp Glu Tyr Glu Leu Leu Cys Pro Asp Asn Thr Arg
                245                 250                 255

Lys Pro Val Asp Lys Phe Lys Asp Cys His Leu Ala Arg Val Pro Ser
            260                 265                 270

His Ala Val Val Ala Arg Ser Val Asn Gly Lys Glu Asp Ala Ile Trp
        275                 280                 285

Asn Leu Leu Arg Gln Ala Gln Glu Lys Phe Gly Lys Asp Lys Ser Pro

290                 295                 300

Lys Phe Gln Leu Phe Gly Ser Pro Ser Gly Gln Lys Asp Leu Leu Phe
305                 310                 315                 320

Lys Asp Ser Ala Ile Gly Phe Ser Arg Val Pro Pro Arg Ile Asp Ser
                325                 330                 335

Gly Leu Tyr Leu Gly Ser Gly Tyr Phe Thr Ala Ile Gln Asn Leu Arg
            340                 345                 350

Lys Ser Glu Glu Glu Val Ala Ala Arg Arg Ala Arg Val Val Trp Cys
        355                 360                 365

Ala Val Gly Glu Gln Glu Leu Arg Lys Cys Asn Gln Trp Ser Gly Leu
    370                 375                 380

Ser Glu Gly Ser Val Thr Cys Ser Ser Ala Ser Thr Thr Glu Asp Cys
385                 390                 395                 400

Ile Ala Leu Val Leu Lys Gly Glu Ala Asp Ala Met Ser Leu Asp Gly
                405                 410                 415

Gly Tyr Val Tyr Thr Ala Gly Lys Cys Gly Leu Val Pro Val Leu Ala
            420                 425                 430

Glu Asn Tyr Lys Ser Gln Gln Ser Ser Asp Pro Asp Pro Asn Cys Val
        435                 440                 445

Asp Arg Pro Val Glu Gly Tyr Leu Ala Val Ala Val Val Arg Arg Ser
    450                 455                 460

Asp Thr Ser Leu Thr Trp Asn Ser Val Lys Gly Lys Lys Ser Cys His
465                 470                 475                 480

Thr Ala Val Asp Arg Thr Ala Gly Trp Asn Ile Pro Met Gly Leu Leu
                485                 490                 495

Phe Asn Gln Thr Gly Ser Cys Lys Phe Asp Glu Tyr Phe Ser Gln Ser
            500                 505                 510

Cys Ala Pro Gly Ser Asp Pro Arg Ser Asn Leu Cys Ala Leu Cys Ile
        515                 520                 525

Gly Asp Glu Gln Gly Glu Asn Lys Cys Val Pro Asn Ser Asn Glu Arg
    530                 535                 540

Tyr Tyr Gly Tyr Thr Gly Ala Phe Arg Cys Leu Ala Glu Asn Ala Gly
545                 550                 555                 560

Asp Val Ala Phe Val Lys Asp Val Thr Val Leu Gln Asn Thr Asp Gly
                565                 570                 575

Asn Asn Asn Glu Ala Trp Ala Lys Asp Leu Lys Leu Ala Asp Phe Ala
            580                 585                 590

Leu Leu Cys Leu Asp Gly Lys Arg Lys Pro Val Thr Glu Ala Arg Ser
        595                 600                 605

Cys His Leu Ala Met Ala Pro Asn His Ala Val Val Ser Arg Met Asp
    610                 615                 620

Lys Val Glu Arg Leu Lys Gln Val Leu Leu His Gln Gln Ala Lys Phe
625                 630                 635                 640

Gly Arg Asn Gly Ser Asp Cys Pro Asp Lys Phe Cys Leu Phe Gln Ser
                645                 650                 655

Glu Thr Lys Asn Leu Leu Phe Asn Asp Asn Thr Glu Cys Leu Ala Arg
            660                 665                 670

Leu His Gly Lys Thr Thr Tyr Glu Lys Tyr Leu Gly Pro Gln Tyr Val
        675                 680                 685

Ala Gly Ile Thr Asn Leu Lys Lys Cys Ser Thr Ser Pro Leu Leu Glu
    690                 695                 700

Ala Cys Glu Phe Leu Arg Lys Asp Pro Asp Ala His Lys Ser Glu Val
705                 710                 715                 720

Ala His Arg Phe Lys Asp Leu Gly Glu Glu Asn Phe Lys Ala Leu Val
                725                 730                 735

Leu Ile Ala Phe Ala Gln Tyr Leu Gln Gln Cys Pro Phe Glu Asp His
            740                 745                 750

Val Lys Leu Val Asn Glu Val Thr Glu Phe Ala Lys Thr Cys Val Ala
        755                 760                 765

Asp Glu Ser Ala Glu Asn Cys Asp Lys Ser Leu His Thr Leu Phe Gly
    770                 775                 780

Asp Lys Leu Cys Thr Val Ala Thr Leu Arg Glu Thr Tyr Gly Glu Met
785                 790                 795                 800

Ala Asp Cys Cys Ala Lys Gln Glu Pro Glu Arg Asn Glu Cys Phe Leu
                805                 810                 815

Gln His Lys Asp Asp Asn Pro Asn Leu Pro Arg Leu Val Arg Pro Glu
            820                 825                 830

Val Asp Val Met Cys Thr Ala Phe His Asp Asn Glu Glu Thr Phe Leu
        835                 840                 845

Lys Lys Tyr Leu Tyr Glu Ile Ala Arg Arg His Pro Tyr Phe Tyr Ala
    850                 855                 860

Pro Glu Leu Leu Phe Phe Ala Lys Arg Tyr Lys Ala Ala Phe Thr Glu
865                 870                 875                 880

Cys Cys Gln Ala Ala Asp Lys Ala Ala Cys Leu Leu Pro Lys Leu Asp
                885                 890                 895

Glu Leu Arg Asp Glu Gly Lys Ala Ser Ser Ala Lys Gln Arg Leu Lys
            900                 905                 910

Cys Ala Ser Leu Gln Lys Phe Gly Glu Arg Ala Phe Lys Ala Trp Ala
        915                 920                 925

Val Ala Arg Leu Ser Gln Arg Phe Pro Lys Ala Glu Phe Ala Glu Val
    930                 935                 940

Ser Lys Leu Val Thr Asp Leu Thr Lys Val His Thr Glu Cys Cys His
945                 950                 955                 960

Gly Asp Leu Leu Glu Cys Ala Asp Asp Arg Ala Asp Leu Ala Lys Tyr
                965                 970                 975

Ile Cys Glu Asn Gln Asp Ser Ile Ser Ser Lys Leu Lys Glu Cys Cys
            980                 985                 990

Glu Lys Pro Leu Leu Glu Lys Ser  His Cys Ile Ala Glu  Val Glu Asn
        995                 1000                1005

Asp Glu  Met Pro Ala Asp Leu  Pro Ser Leu Ala Ala  Asp Phe Val
    1010                1015                1020

Glu Ser  Lys Asp Val Cys Lys  Asn Tyr Ala Glu Ala  Lys Asp Val
    1025                1030                1035

Phe Leu  Gly Met Phe Leu Tyr  Glu Tyr Ala Arg Arg  His Pro Asp
    1040                1045                1050

Tyr Ser Val Val Leu Leu Leu Arg Leu Ala Lys Thr Tyr Glu Thr
    1055                1060                1065

Thr Leu Glu Lys Cys Cys Ala Ala Ala Asp Pro His Glu Cys Tyr
    1070                1075                1080

Ala Lys Val Phe Asp Glu Phe Lys Pro Leu Val Glu Glu Pro Gln
    1085                1090                1095

Asn Leu Ile Lys Gln Asn Cys Glu Leu Phe Glu Gln Leu Gly Glu
    1100                1105                1110

Tyr Lys Phe Gln Asn Ala Leu Leu Val Arg Tyr Thr Lys Lys Val
    1115                1120                1125

Pro Gln Val Ser Thr Pro Thr Leu Val Glu Val Ser Arg Asn Leu
    1130                1135                1140

Gly Lys Val Gly Ser Lys Cys Cys Lys His Pro Glu Ala Lys Arg
    1145                1150                1155

Met Pro Cys Ala Glu Asp Tyr Leu Ser Val Val Leu Asn Gln Leu
    1160                1165                1170

Cys Val Leu His Glu Lys Thr Pro Val Ser Asp Arg Val Thr Lys
    1175                1180                1185

Cys Cys Thr Glu Ser Leu Val Asn Arg Arg Pro Cys Phe Ser Ala
    1190                1195                1200

Leu Glu Val Asp Glu Thr Tyr Val Pro Lys Glu Phe Asn Ala Glu
    1205                1210                1215

Thr Phe Thr Phe His Ala Asp Ile Cys Thr Leu Ser Glu Lys Glu
    1220                1225                1230

Arg Gln Ile Lys Lys Gln Thr Ala Leu Val Glu Leu Val Lys His
    1235                1240                1245

Lys Pro Lys Ala Thr Lys Glu Gln Leu Lys Ala Val Met Asp Asp
    1250                1255                1260

Phe Ala Ala Phe Val Glu Lys Cys Cys Lys Ala Asp Asp Lys Glu
    1265                1270                1275

Thr Cys Phe Ala Glu Glu Gly Lys Lys Leu Val Ala Ala Ser Gln

1280                1285                1290

Ala Ala  Leu Gly Leu
    1295

<210>  11
<211>  890
<212>  PRT
<213>  Artificial Sequence

<220>
<223>  hLF-G-CSF fusion protein

<400>  11

Met Lys Leu Val Phe Leu Val Leu Leu Phe Leu Gly Ala Leu Gly Leu
1               5                   10                  15

Cys Leu Ala Gly Arg Arg Arg Arg Ser Val Gln Trp Cys Ala Val Ser
            20                  25                  30

Gln Pro Glu Ala Thr Lys Cys Phe Gln Trp Gln Arg Asn Met Arg Lys
        35                  40                  45

Val Arg Gly Pro Pro Val Ser Cys Ile Lys Arg Asp Ser Pro Ile Gln
    50                  55                  60

Cys Ile Gln Ala Ile Ala Glu Asn Arg Ala Asp Ala Val Thr Leu Asp
65                  70                  75                  80

Gly Gly Phe Ile Tyr Glu Ala Gly Leu Ala Pro Tyr Lys Leu Arg Pro
                85                  90                  95

Val Ala Ala Glu Val Tyr Gly Thr Glu Arg Gln Pro Arg Thr His Tyr
            100                 105                 110

Tyr Ala Val Ala Val Val Lys Lys Gly Gly Ser Phe Gln Leu Asn Glu
        115                 120                 125

Leu Gln Gly Leu Lys Ser Cys His Thr Gly Leu Arg Arg Thr Ala Gly
    130                 135                 140

Trp Asn Val Pro Ile Gly Thr Leu Arg Pro Phe Leu Asn Trp Thr Gly
145                 150                 155                 160

Pro Pro Glu Pro Ile Glu Ala Ala Val Ala Arg Phe Phe Ser Ala Ser
                165                 170                 175

Cys Val Pro Gly Ala Asp Lys Gly Gln Phe Pro Asn Leu Cys Arg Leu
            180                 185                 190

Cys Ala Gly Thr Gly Glu Asn Lys Cys Ala Phe Ser Ser Gln Glu Pro
        195                 200                 205

Tyr Phe Ser Tyr Ser Gly Ala Phe Lys Cys Leu Arg Asp Gly Ala Gly
    210                 215                 220

Asp Val Ala Phe Ile Arg Glu Ser Thr Val Phe Glu Asp Leu Ser Asp
225                 230                 235                 240

Glu Ala Glu Arg Asp Glu Tyr Glu Leu Leu Cys Pro Asp Asn Thr Arg
                245                 250                 255

Lys Pro Val Asp Lys Phe Lys Asp Cys His Leu Ala Arg Val Pro Ser
            260                 265                 270

His Ala Val Val Ala Arg Ser Val Asn Gly Lys Glu Asp Ala Ile Trp
        275                 280                 285

Asn Leu Leu Arg Gln Ala Gln Glu Lys Phe Gly Lys Asp Lys Ser Pro
    290                 295                 300

Lys Phe Gln Leu Phe Gly Ser Pro Ser Gly Gln Lys Asp Leu Leu Phe
305                 310                 315                 320

Lys Asp Ser Ala Ile Gly Phe Ser Arg Val Pro Pro Arg Ile Asp Ser
                325                 330                 335

Gly Leu Tyr Leu Gly Ser Gly Tyr Phe Thr Ala Ile Gln Asn Leu Arg
            340                 345                 350

Lys Ser Glu Glu Glu Val Ala Ala Arg Arg Ala Arg Val Val Trp Cys
        355                 360                 365

Ala Val Gly Glu Gln Glu Leu Arg Lys Cys Asn Gln Trp Ser Gly Leu
    370                 375                 380

Ser Glu Gly Ser Val Thr Cys Ser Ser Ala Ser Thr Thr Glu Asp Cys
385                 390                 395                 400

Ile Ala Leu Val Leu Lys Gly Glu Ala Asp Ala Met Ser Leu Asp Gly
                405                 410                 415

Gly Tyr Val Tyr Thr Ala Gly Lys Cys Gly Leu Val Pro Val Leu Ala
            420                 425                 430

Glu Asn Tyr Lys Ser Gln Gln Ser Ser Asp Pro Asp Pro Asn Cys Val

435                 440                 445

Asp Arg Pro Val Glu Gly Tyr Leu Ala Val Ala Val Val Arg Arg Ser
    450                 455                 460

Asp Thr Ser Leu Thr Trp Asn Ser Val Lys Gly Lys Lys Ser Cys His
465                 470                 475                 480

Thr Ala Val Asp Arg Thr Ala Gly Trp Asn Ile Pro Met Gly Leu Leu
                485                 490                 495

Phe Asn Gln Thr Gly Ser Cys Lys Phe Asp Glu Tyr Phe Ser Gln Ser
            500                 505                 510

Cys Ala Pro Gly Ser Asp Pro Arg Ser Asn Leu Cys Ala Leu Cys Ile
        515                 520                 525

Gly Asp Glu Gln Gly Glu Asn Lys Cys Val Pro Asn Ser Asn Glu Arg
    530                 535                 540

Tyr Tyr Gly Tyr Thr Gly Ala Phe Arg Cys Leu Ala Glu Asn Ala Gly
545                 550                 555                 560

Asp Val Ala Phe Val Lys Asp Val Thr Val Leu Gln Asn Thr Asp Gly
                565                 570                 575

Asn Asn Asn Glu Ala Trp Ala Lys Asp Leu Lys Leu Ala Asp Phe Ala
            580                 585                 590

Leu Leu Cys Leu Asp Gly Lys Arg Lys Pro Val Thr Glu Ala Arg Ser
        595                 600                 605

Cys His Leu Ala Met Ala Pro Asn His Ala Val Val Ser Arg Met Asp
    610                 615                 620

Lys Val Glu Arg Leu Lys Gln Val Leu Leu His Gln Gln Ala Lys Phe
625                 630                 635                 640

Gly Arg Asn Gly Ser Asp Cys Pro Asp Lys Phe Cys Leu Phe Gln Ser
                645                 650                 655

Glu Thr Lys Asn Leu Leu Phe Asn Asp Asn Thr Glu Cys Leu Ala Arg
            660                 665                 670

Leu His Gly Lys Thr Thr Tyr Glu Lys Tyr Leu Gly Pro Gln Tyr Val
        675                 680                 685

Ala Gly Ile Thr Asn Leu Lys Lys Cys Ser Thr Ser Pro Leu Leu Glu
    690                 695                 700

Ala Cys Glu Phe Leu Arg Lys Ser Arg Leu Glu Ala Thr Pro Leu Gly
705                 710                 715                 720

Pro Ala Ser Ser Leu Pro Gln Ser Phe Leu Leu Lys Cys Leu Glu Gln
                725                 730                 735

Val Arg Lys Ile Gln Gly Asp Gly Ala Ala Leu Gln Glu Lys Leu Cys
            740                 745                 750

Ala Thr Tyr Lys Leu Cys His Pro Glu Glu Leu Val Leu Leu Gly His
        755                 760                 765

Ser Leu Gly Ile Pro Trp Ala Pro Leu Ser Ser Cys Pro Ser Gln Ala
    770                 775                 780

Leu Gln Leu Ala Gly Cys Leu Ser Gln Leu His Ser Gly Leu Phe Leu
785                 790                 795                 800

Tyr Gln Gly Leu Leu Gln Ala Leu Glu Gly Ile Ser Pro Glu Leu Gly
                805                 810                 815

Pro Thr Leu Asp Thr Leu Gln Leu Asp Val Ala Asp Phe Ala Thr Thr
            820                 825                 830

Ile Trp Gln Gln Met Glu Glu Leu Gly Met Ala Pro Ala Leu Gln Pro
        835                 840                 845

Thr Gln Gly Ala Met Pro Ala Phe Ala Ser Ala Phe Gln Arg Arg Ala
    850                 855                 860

Gly Gly Val Leu Val Ala Ser His Leu Gln Ser Phe Leu Glu Val Ser
865                 870                 875                 880

Tyr Arg Val Leu Arg His Leu Ala Gln Pro
                885                 890